# EUROPEAN PATENT APPLICATION

(11) **EP 4 480 494 A1**
(43) Date of publication of application: **25.12.2024**
(21) Application number: 23756416.6
(22) Date of filing: 15.02.2023
(51) Int. Cl.: A61K 45/00

(54) **COMPOSITION FOR USE IN TREATMENT OF CANCER, INFLAMMATORY DISEASES, OR OBESITY, AND USE THEREOF**

(30) Priority: 16.02.2022 JP 2022021824
(71) Applicant: National University Corporation Tokyo Medical and Dental University, Tokyo 113-8510 (JP)
(72) Inventor: ASAHARA Hiroshi, Tokyo 113-8510 (JP); UCHIDA Yutaro, Tokyo 113-8510 (JP); KURIMOTO Ryota, Tokyo 113-8510 (JP); CHIBA Tomoki, Tokyo 113-8510 (JP)
(74) Representative: Plasseraud IP
(86) International application number: PCT/JP2023/005320
(87) International publication number: WO 2023/157891

(57) **Abstract**

One of the objectives of the present invention is to provide an agent for use in the treatment of triple negative breast cancer, a blood marker for triple negative breast cancer, and an agent for use in the treatment of inflammatory diseases or obesity. By employing an inhibitor of the ZCCHC24 protein or an agent that suppresses the expression of the ZCCHC24 gene as an active ingredient, a pharmaceutical composition for use in the treatment of triple negative breast cancer, inflammatory diseases, or obesity can be manufactured. An inhibitor of the ZCCHC24 protein or an agent that suppresses the ZCCHC24 gene expression can also be used in combination with a BET inhibitor.

## Description

### Technical Field

The present invention relates to compositions for use in the treatment of cancer, inflammatory disease, or obesity. More specifically, it relates to compositions for use in the treatment of cancer, inflammatory disease, or obesity, in particular, triple negative breast cancer, which comprise as an active ingredient an inhibitor of the ZCCHC24 protein or an agent that inhibits the expression of the ZCCHC24 gene.

### Background Art

Breast cancer is one of the most common cancers in women, and triple negative breast cancer (TNBC) accounts for approximately 20% of all breast cancers. Due to the high degree of malignancy biologically, an extremely high recurrence rate within 3 years, and the significantly shorter period of survival after recurrence compared to other types, there has been a search for its treatment methods. Hormone therapy and molecular target drugs targeting HER2, which are used to treat other types of breast cancer, are ineffective, and there is a strong need for the development of new treatments based on elucidation of the pathology of TNBC.

What makes triple negative breast cancer particularly difficult to treat is the presence of a cancer stem cell-like population (Cancer stem-like population). Among cancer cell populations, this cancer stem cell-like population has particularly high resistance to drugs, and is a population with high tumorigenicity, which results in resistance to treatment. In the past, CD44, ALDH1, NRP1, CD 13 3 and such have been reported as cell surface markers for this cancer stem cell-like population. In addition, ZEB1, ID2, NFκB and such have been reported as transcriptional regulators that control this cancer stem cell-like population. Furthermore, looking at other types of cancer, it has been reported that RNA-binding proteins such as Musashi-2 regulate the expression in pancreatic cancer and brain tumors in order to maintain the stemness of cancer stem cells. However, the gene network, including the RNA hierarchy, in breast cancer stem cell-like populations has not been elucidated.

PDL1 is one of the particularly important effector molecules in this breast cancer stem cell-like population. It has been reported in recent years that the expression of CD44, which is particularly important as a breast cancer stem cell-like marker (Breast cancer stem like marker), is essential for maintaining the PDL1 expression, and that the expression of PDL1 is extremely high in the cancer stem cell-like fraction. Antibody therapy using immune checkpoint inhibitors targeting PDL1 and PD-1, a receptor on the T cell side, is being investigated at the clinical level for triple negative breast cancer, and the results of preoperative treatment of TNBC with atezolizumab which targets PDL1 have been reported. However, the functional analysis in breast cancer is still insufficient, and further accumulation of evidence is urgently needed. In particular, it is clear that deletion of the 3' untranslated region (3' UTR) is observed in many tumors as a mechanism for regulating the expression of PDL1, and that this region is suppressed and controlled by TTP, which is an RNA binding protein. However, the RNA-level gene network that maintains the PDL1 expression is still unknown.

### Citation List

### Non-Patent Documents

Patent Document 1: WO2017/151554A1
Patent Document 2: WO2020/104777A1

Non-Patent Document 1: Bianchini G, Balko JM, Mayer IA, Sanders ME, Gianni L. Triple negative breast cancer: challenges and opportunities of a heterogeneous disease. Nat Rev Clin Oncol. 2016;13(11):674-90
Non-Patent Document 2: Marra A, Trapani D, Viale G, Criscitiello C, Curigliano G. Practical classification of triple negative breast cancer: intratumoral heterogeneity, mechanisms of drug resistance, and novel therapies. NPJ Breast Cancer. 2020;6:54
Non-Patent Document 3: Honeth G, Bendahl PO, Ringner M, Saal LH, Gruvberger-Saal SK, Lovgren K, et al. The CD44+/CD24- phenotype is enriched in basal-like breast tumors. Breast Cancer Res. 2008;10(3):R53.
Non-Patent Document 4: Liu TJ, Sun BC, Zhao XL, Zhao XM, Sun T, Gu Q, et al. CD133+ cells with cancer stem cell characteristics associates with vasculogenic mimicry in triple negative breast cancer. Oncogene. 2013;32(5):544-53
Non-Patent Document 5: Ma F, Li H, Wang H, Shi X, Fan Y, Ding X, et al. Enriched CD44(+)/CD24(-) population drives the aggressive phenotypes presented in triple negative breast cancer (TNBC). Cancer Lett. 2014;353(2):153-9
Non-Patent Document 6: Tominaga K, Minato H, Murayama T, Sasahara A, Nishimura T, Kiyokawa E, et al. Semaphorin signaling via MICAL3 induces symmetric cell division to expand breast cancer stem-like cells. Proc Natl Acad Sci USA. 2019;116(2):625-30

### Summary of the Invention

### Problems to be Solved by the Invention

One of the objectives of the present invention is to provide a composition for use in the treatment of cancer, inflammatory disease, or obesity, in particular, triple negative breast cancer (TNBC).

### Means for Solving the Problems

As an attempt to identify RNA-binding proteins that control cancer stemness, the present inventors focused on the expression of PDL1, and performed RNA-binding protein screening using reporter cells labeled with the PDL1 protein expression by HiBiT tags. As a result, the present inventors succeeded in identifying ZCCHC24, an RNA-binding protein with unknown function, and further found that ZCCHC24 is strongly localized in the breast cancer stem cell-like fraction, which broadly characterizes cancer stemness and contributes to tumorigenesis. Then, a TNBC therapeutic drug that targets this ZCCHC24 was successfully identified. In other words, the present inventors discovered that an inhibitor of the ZCCHC24 protein or an agent that suppresses the expression of the ZCCHC24 gene can be used as a therapeutic drug for TNBC, and identified a Wee1/Chk1 inhibitor as a specific drug that suppresses the expression of the ZCCHC24 gene.

The present inventors also discovered a novel breast cancer stem cell fraction (hereinafter also referred to as "Stem II") in which ZCCHC24 shows a particularly high expression, and identified NRP1(+) NCAM1(-) in combination with the cell surface markers that define this Stem II cell fraction. Furthermore, it was found that this Stem II population has high tumorigenicity and owns the characteristics of breast cancer stem cells, and that ZCCHC24 is important for maintaining the Stem II population and maintenance of tumorigenicity. In other words, the present inventors have identified a novel cancer stem cell population (Stem II population) as a therapeutic target for the TNBC therapeutic drug of the present invention that targets ZCCHC24.

Furthermore, the present inventors have found that inhibitors of the ZCCHC24 protein or agents that suppress the expression of the ZCCHC24 gene are effective for improvement of inflammatory disease and obesity.

The present invention is based on such findings and encompasses the embodiments below.
[1] A composition for use in the treatment of triple negative breast cancer, comprising an inhibitor of the ZCCHC24 protein or an agent that suppresses the expression of the ZCCHC24 gene as an active ingredient for use in the treatment of a patient having a NRP1 (+) NCAM1 (-) cell fraction.
[2] The composition for use in the treatment of triple negative breast cancer according to [1], wherein the agent that suppresses the expression of the ZCCHC24 gene is a Wee1/Chk1 inhibitor.
[3] The composition for use in the treatment of triple negative breast cancer according to [1] or [2], for use in combination with a BET inhibitor.
[4] The composition for use in the treatment of triple negative breast cancer according to any one of [1] to [3], wherein the inhibitor of the ZCCHC24 protein is selected from the group consisting of neutralizing antibodies, nucleic acid drugs, and low molecular weight compounds.
[5] The composition for use in the treatment of triple negative breast cancer according to any one of [2] to [4], wherein the Wee1/Chk1 inhibitor is selected from the group consisting of PD407824, AZD-1775, ZN-c3, Debio-0123, IMP-7068, GDC-0575, ESP-01, PNT-737, BEBT-260, AZD7762, LY2603618, MK- 8776, CHIR-124, PF-477736, roscovitine, SNS-032, dinaciclib, flavopiridol, AT7519, purvalanol A, RO-3306, SU9516, XL413, NU6027, P276-00, AZD5438, PHA-793887, JNJ-7706621 , BMS-265246, milciclib, MK-8776, R547, and adavosertib.
[6] The composition for use in the treatment of triple negative breast cancer according to any one of [1] to [5], further comprising a BET inhibitor.
[7] The composition for use in the treatment of triple negative breast cancer according to [6], wherein the BET inhibitor is selected from the group consisting of JQ1, pelabresib, BMS-986158, INCB-057643, ODM-207, PLX-2853, ABBV-744, BI-894999, BPI-23314, CC-90010, FT-1101, JAB- 8263, mibebresib, SF-1126, and SYHA-1801.
[8] The composition for use in the treatment of triple negative breast cancer according to [5], wherein the Wee1/Chk1 inhibitor is selected from the group consisting of PD407824, AZD-1775, ZN-c3, Debio-0123, IMP-7068, GDC-0575, ESP-01, PNT-737, BEBT-260, AZD7762, LY2603618, MK- 8776, CHIR-124, PF-477736, rosovitin, SNS-032, dinaciclib, flavopiridol, AT7519, purvalanol A, RO-3306, SU9516, XL413, NU6027, P276-00, AZD5438, PHA-793887, JNJ-7706621 , BMS-265246, MK-8776, and R547.
[9] The composition for use in the treatment of triple negative breast cancer according to [1], wherein the agent that suppresses the expression of the ZCCHC24 gene is a nucleic acid drug.
[10] The composition for use in the treatment of triple negative breast cancer according to [1], wherein the inhibitor of the ZCCHC24 protein comprises a 19-25 base long miRNA having a sequence with a full-length GC content of 40-60%, and consists of:
   (i) a seed sequence complementary to at least a portion of WGUWHWWA;
   (ii) an adenine or a uracil base on the 5' side of the seed sequence; and
   (iii) a scrambled sequence on the 3' side of the seed sequence.
[11] A composition for use in the treatment of triple negative breast cancer, comprising as an active ingredient an inhibitor of the ZCCHC24 protein or an agent that suppresses the expression of the ZCCHC24 gene (with the proviso that milciclib and adavosertib are excludded).
[12] A composition for use in the treatment of triple negative breast cancer, comprising as an active ingredient an inhibitor of the ZCCHC24 protein or a nucleic acid drug that suppresses the expression of the ZCCHC24 gene.
[13] A composition for use in the treatment of triple negative breast cancer to be used in combination with a BET inhibitor, comprising an inhibitor of the ZCCHC24 protein or an agent that suppresses the expression of the ZCCHC24 gene as an active ingredient.
[14] A composition for use in the treatment of triple negative breast cancer, comprising as an active ingredient an inhibitor of the ZCCHC24 protein or an agent that suppresses the expression of the ZCCHC24 gene, wherein the agent that suppresses the expression of the ZCCHC24 gene is a Wee1/Chk1 inhibitor, and wherein the Wee1/Chk1 inhibitor is selected from the group consisting of PD407824, AZD-1775, ZN-c3, Debio-0123, IMP-7068, GDC-0575, ESP-01, PNT-737, BEBT-260, AZD7762, LY2603618, MK-8776, CHIR-124, PF-477736, rosovitin, SNS-032, dinaciclib, flavopiridol, AT7519, purvalanol A, RO-3306, SU9516, XL413, NU6027, P276-00, AZD5438, PHA-793887, JNJ-7706621, BMS-265246, MK-8776, and R547.
[15] A method for testing breast cancer in a test subject, comprising:
   a) measuring the amount of the ZCCHC24 protein in a body fluid sample derived from the test subject; and
   b) comparing the amount of the ZCCHC24 protein with a predetermined reference value, wherein the test method indicates the possibility of breast cancer based on that the amount of the ZCCHC24 protein is higher than the predetermined reference value.
[16] A method for testing breast cancer in a test subject, comprising determining whether or not the cell sample derived from the test subject has a NRP1 (+) NCAM1 (-) cell fraction, wherein the test method indicates the possibility of breast cancer based on the presence of the subcellular fraction.
[17] A method for examining the condition of breast cancer in a test subject, comprising determining whether or not the cell sample derived from the test subject has a NRP1 (+) NCAM1 (-) cell fraction, wherein the test method determines that the breast cancer has a high degree of malignancy when it has the subcellular fraction.
[18] A method for examining the condition of breast cancer in a test subject, comprising determining whether or not the cell sample derived from the test subject has a NRP1 (+) NCAM1 (-) cell fraction, wherein the test method identifies the test subject to be eligible for administration of a composition for use in the treatment of triple negative breast cancer when it has the cell fraction.
[19] A composition for use in the treatment of an inflammatory disease, comprising as an active ingredient an inhibitor of the ZCCHC24 protein or an agent that suppresses the expression of the ZCCHC24 gene.
[20] The composition for use in the treatment of an inflammatory disease according to [19], wherein the agent that suppresses the expression of the ZCCHC24 gene is a Wee1/Chk1 inhibitor.
[21] The composition for use in the treatment of an inflammatory disease according to [19], wherein the agent that suppresses the expression of the ZCCHC24 gene is a nucleic acid drug.
[22] The composition for use in the treatment of an inflammatory disease according to [19], wherein the inhibitor of the ZCCHC24 protein comprises a 19-25 base long miRNA having a sequence with a full-length GC content of 40-60%, and consists of:
   (i) a seed sequence complementary to at least a portion of WGUWHWWA;
   (ii) an adenine or a uracil base on the 5' side of the seed sequence; and
   (iii) a scrambled sequence on the 3' side of the seed sequence.
[23] A composition for use in the treatment of obesity, comprising as an active ingredient an inhibitor of the ZCCHC24 protein or an agent that suppresses the expression of the ZCCHC24 gene.
[24] The composition for use in the treatment of obesity according to [23], wherein the agent that suppresses the expression of the ZCCHC24 gene is a Wee1/Chk1 inhibitor.
[25] The composition for use in the treatment of obesity according to [23], wherein the agent that suppresses the expression of the ZCCHC24 gene is a nucleic acid drug.
[26] The composition for use in the treatment of obesity according to [23], wherein the inhibitor of the ZCCHC24 protein comprises a 19-25 base long miRNA having a sequence with a full-length GC content of 40-60%, and consists of:
   (i) a seed sequence complementary to at least a portion of WGUWHWWA;
   (ii) an adenine or a uracil base on the 5' side of the seed sequence; and
   (iii) a scrambled sequence on the 3' side of the seed sequence.
[27] A method of screening for agents for use in the treatment of triple negative breast cancer, inflammatory disease, or obesity, wherein the method comprises:
   (i) a step of preparing cells expressing ZCCHC24;
   (ii) a step of contacting a test substance with the cells;,
   (iii) a step of measuring the expression level of ZCCHC24; and
   (iv) a step of selecting a substance that reduces the expression level of ZCCHC24.
[28] A method for treating and/or preventing triple negative breast cancer, inflammatory disease, or obesity in a subject in need thereof, wherein the method comprises administering to the subject an effective amount of an inhibitor of the ZCCHC24 protein or an agent that suppresses the expression of the ZCCHC24 gene.
[29] Use of an inhibitor of the ZCCHC24 protein or an agent that suppresses the expression of the ZCCHC24 gene in the manufacture of a medicament for the treatment and/or prevention of triple negative breast cancer, inflammatory disease, or obesity.

### Brief Description of the Drawings

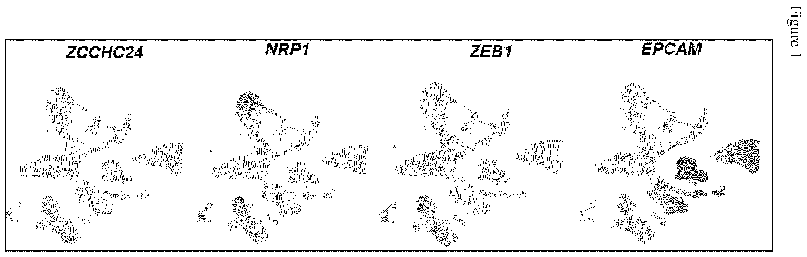

[Figure 1] As a result of re-analysis of human breast cancer specimen scRNA-seq data, ZCCHC24 was identified as a novel RNA-binding protein with unknown function that shows a high expression in cell fractions exhibiting breast cancer stem cell-like expression.

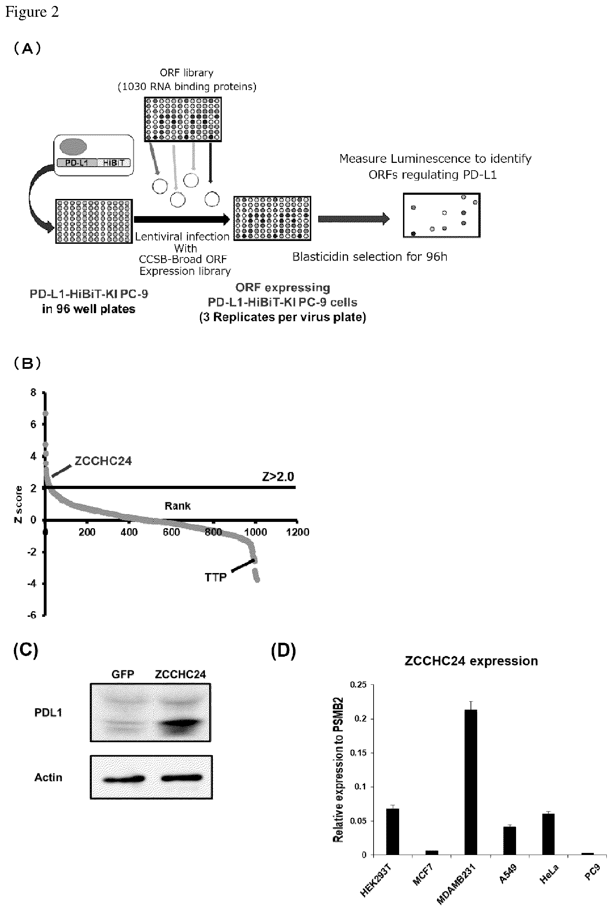

[Figure 2-1] As a result of genetic screening for RNA binding proteins using the PC9-KI cell line in which the HiBiT tag was knocked into the C terminus of PDL1, ZCCHC24 was identified as a gene that increases the expression of PDL1 (Figure 2A, 2B), and the increased expression was also confirmed by Western blotting (Figure 2C). Furthermore, in a comparison among multiple cell lines, the triple negative breast cancer cell line MDAMB231 showed a high expression of ZCCHC24 (Figure 2D).
[Figure 2-2] Furthermore, knockdown of ZCCHC24 reduced the expressions of both PDL1 mRNA and protein (Figure 2E, Figure 2F).

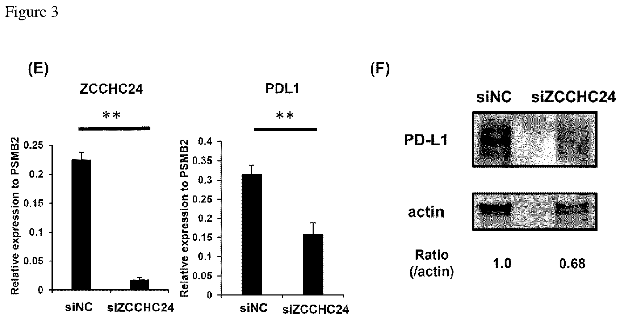

[Figure 3] Re-analysis of single cell RNA seq data for 5 human breast cancer specimens in Wu et al, EMBO J. 2020 revealed that the cell fractions were divided into 23 clusters (Figure 3A), and ZCCHC24 has a tendency to be specifically expressed in cluster 9 as revealed by violin plot (Figure 3B). In this cluster 9, vimentin, which greatly contributes to epithelial-mesenchymal transition (EMT) and maintenance of cancer stem cells, was strongly expressed (Figure 3C), and cancer stem cell markers including CD44 high, CD24 negative, NRP1 positive, and ZEB1 positive were shown. It was suggested that ZCCHC24 is strongly expressed in the cancer stem cell-like fraction (Figure 3D).

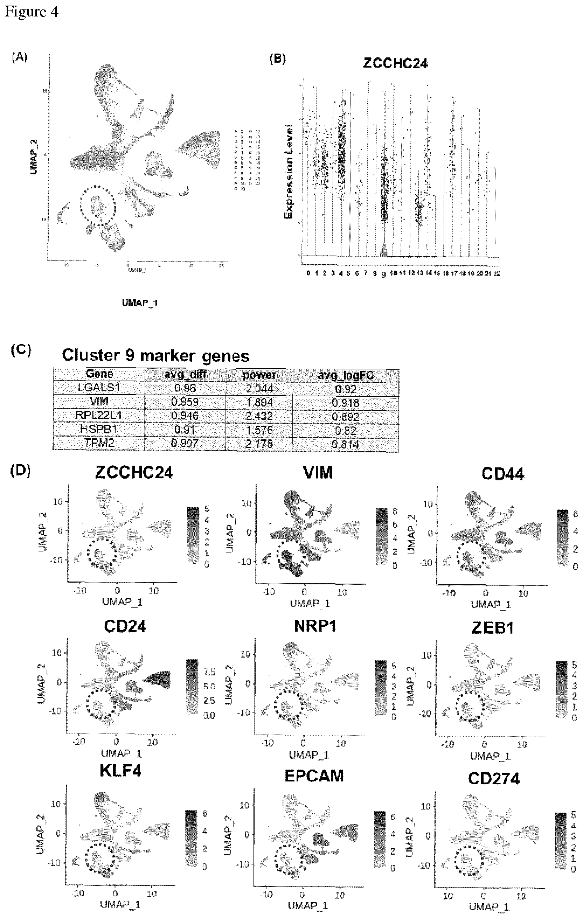

[Figure 4] As a result of the RNA-Seq/qPCR analysis of the human breast cancer cell line MDAMB231, the expression of genes that characterize cancer stemness in breast cancer, in particular, CD44, NRP1, and ZEB 1, were significantly reduced by knockdown of ZCCHC24. Further, it was also confirmed by qPCR that the expression levels of CD44, NRP1, and ZEB1 in human specimen cells derived from breast cancer patients (PDX) were reduced by knockdown of ZCCHC24.

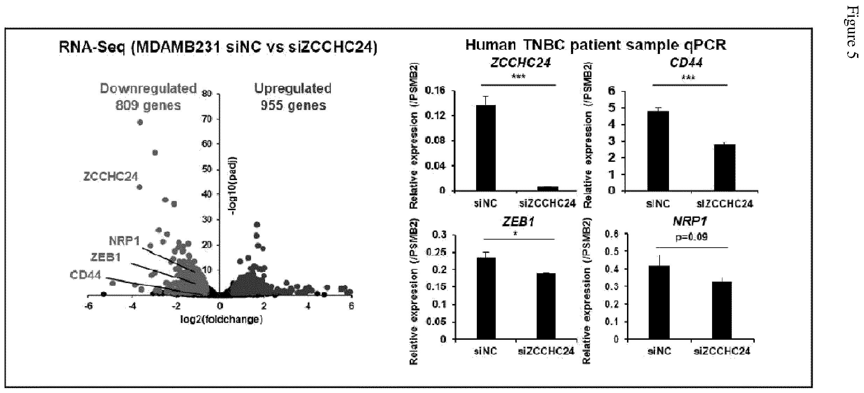

[Figure 5] As a result of RNA-Seq on a cell population in which ZCCHC24 was knocked down, it was revealed that the expressions of the differentially expressed genes (DEGs), a group of genes that characterize cancer stemness in breast cancer, PDL1, and cytokines and chemokines that are important for cancer survival, were significantly down-regulated in ZCCHC24 knockdown cells (Figure 5A). A FACS analysis of MDAMB231 revealed that knockdown of ZCCHC24 reduced the proportion of breast cancer stem cell fraction defined by CD44 positive, NRP1 positive (Figure 5B, Figure 5C). Furthermore, in HCC38, another triple negative breast cancer cell line, the knockdown of ZCCHC24 reduced the expression of cancer stem cell-like cell surface markers such as CD44 and NRP1 at the mRNA level (Figure 5D), and FACS also showed that the CD44 positive, NRP1 positive cell fraction was significantly reduced by knockdown of ZCCHC24 (Figure 5E, Figure 5F).

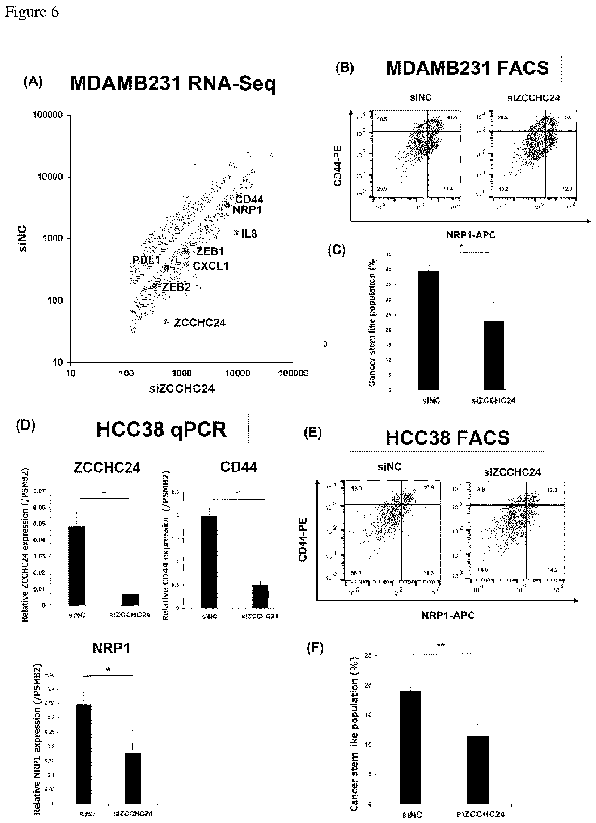

[Figure 6] By merging the eCLIP-Seq results for cells forced to express ZCCHC24 with the RNA-Seq DEGs, 364 genes were identified as target genes whose expression is regulated by direct binding of ZCCHC24 (Figure 6A). When the binding regions in these target genes were classified by RSeqC, it was revealed that ZCCHC24 binds to the 3' UTR region and CDS (Figure 6B), and peak pattern analysis of these target genes revealed that ZCCHC24 directly binds to a group of genes such as PDL1, IL6, IL8, CXCL1, CD44, and NRP1 at the 3' UTR as target genes (Figure 6C). In addition, as a result of the GO term analysis and pathway analysis of these target genes, it was revealed that they are a group of genes that have the functions of negative regulation of the apoptotic process and regulation of cell proliferation, as well as control of pathways in the regulation of focal adhesion and actin cytoskeleton (Figure 6D, Figure 6E).

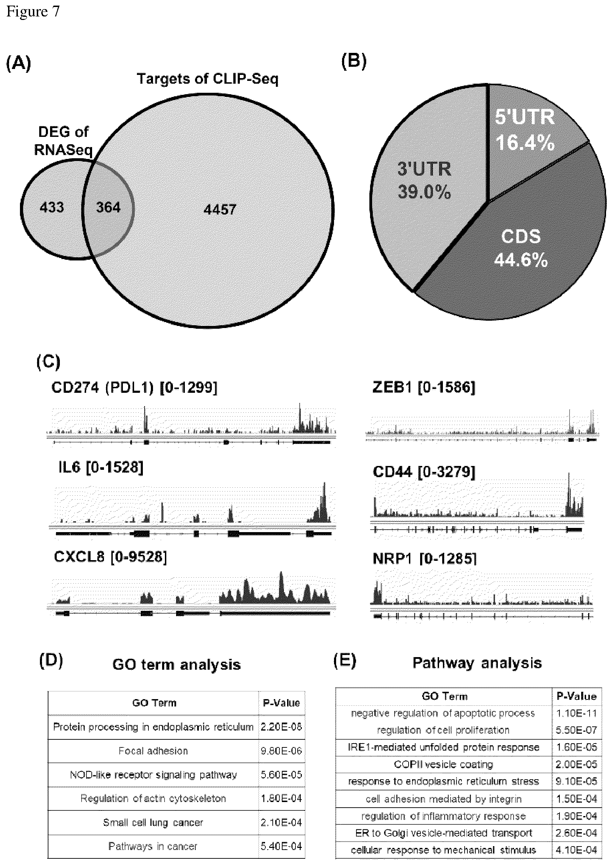

[Figure 7] As a result of the PAR-CLIP analysis of MDAMB231, it was revealed that ZCCHC24 recognizes a novel unique motif sequence of (A/U)GU(A/U)U(A/U)U, and binds to the 3' UTR of the mRNA of the gene group that characterizes cancer stemness such as ZEB 1, CD44, and NRP1.

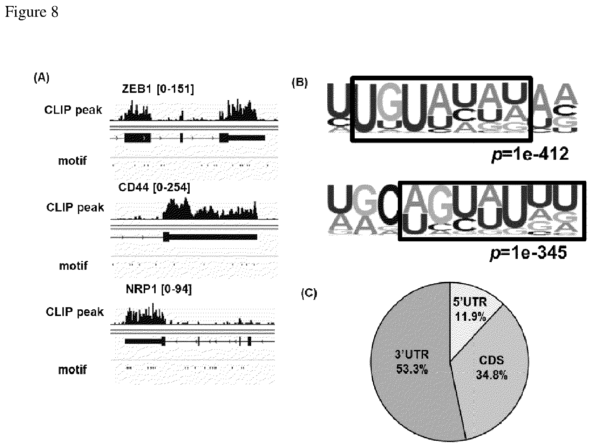

[Figure 8] As a result of the BRIC-Seq analysis of MDAMB231, it was shown that knockdown of ZCCHC24 tends to significantly reduce the stability of RNA of genes that characterize breast cancer stemness, such as ZEB1, CD44, and NRP1.

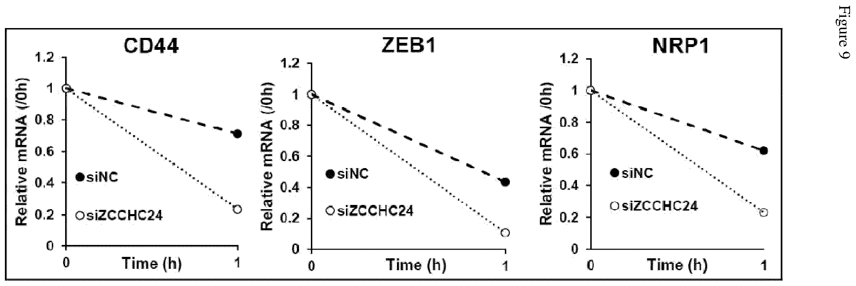

[Figure 9] As a result of the actinomycin D test, it was revealed that knockdown of ZCCHC24 reduced the stability of mRNA of target genes such as CD274 (PDL1), CXCL8 (IL8), IL6, CD44, NRP1, and ZEB1 in MDAMB231 (Figure 9A), and in HCC38, it was also found that the stability of CD44 and NRP1 mRNA was decreased (Figure 9B).

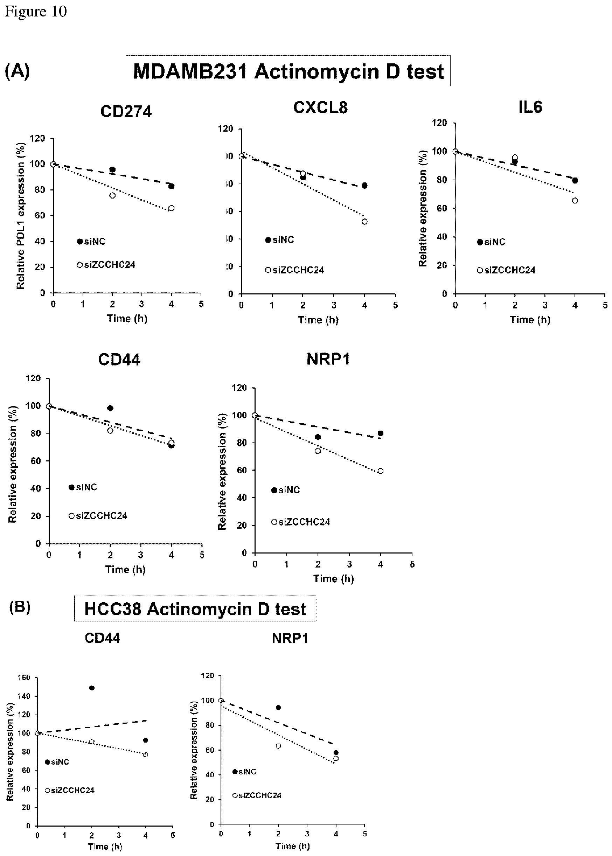

[Figure 10] As a result of the sphere formation assay using MDAMB231, knockdown of ZCCHC24 significantly reduced sphere formation ability (Figure 10A, Figure 10B).

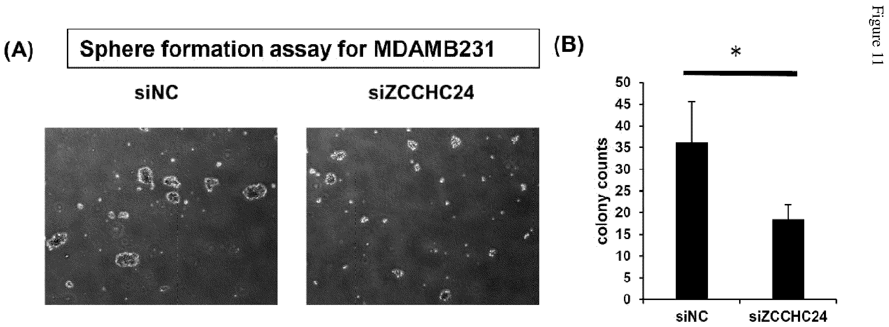

[Figure 11] As a result of the extreme dilution assays (ELDA) for MDAMB231 and HCC38, it was revealed that knockdown of ZCCHC24 tends to reduce the number of breast cancer stem cells per cell count.

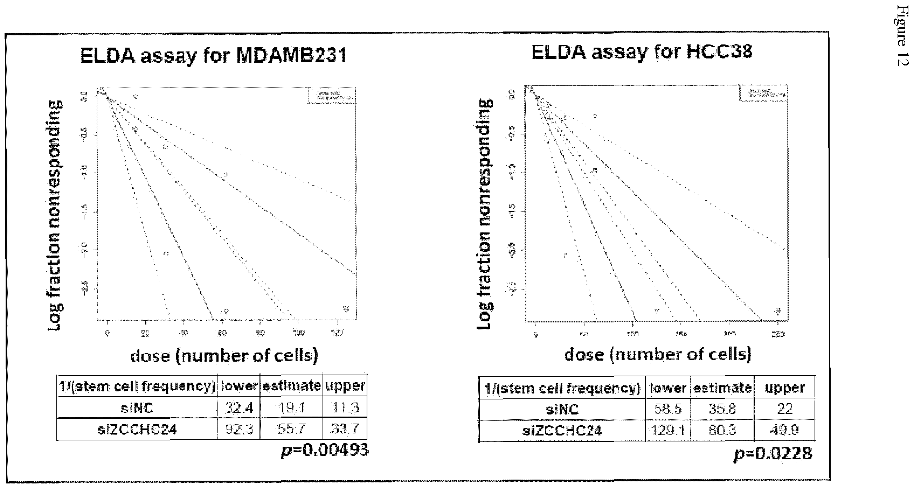

[Figure 12] As a result of the in vivo tumor formation assays in MDAMB231 and the PDX subcutaneous transplantation model, it was revealed that knockdown of ZCCHC24 knockdown tends to significantly reduce tumorigenicity.

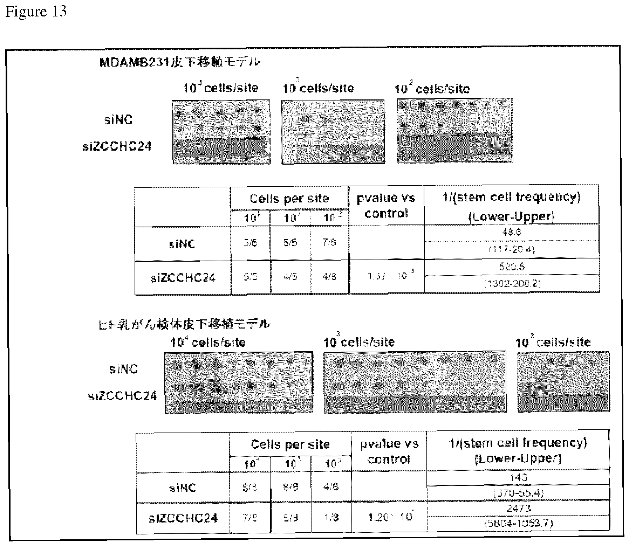

[Figure 13A] As a result of a single-cell RNA-seq analysis of the PDX subcutaneous transplantation model, it was shown that ZCCHC24 knockdown significantly reduced the stem cell-like population exhibiting the cancer stem cell-like expression of NRP1 (+) ZEB1 (+) CD24 (-) EPCAM (-). Furthermore, among the two major grouped cell populations, it was found that the cell fraction Stem II, which is positive for genes such as ZEB1 and NRP1 along with ZCCHC24, was significantly reduced (Figure 13A).
[Figure 13B] Furthermore, in the scRNA analysis of patient-derived breast cancer specimens in the NOG mouse subcutaneous transplantation model, the fractions of NRP1 (+) ZEB1 (+) CD24 (-) EPCAM (-) were broadly divided into two, and among them, it was confirmed that ZCCHC24 was specifically expressed in the NRP1 (+) ZEB1 (+) NCAM1 (-) fraction (Stem II fraction) (Figure 13B).

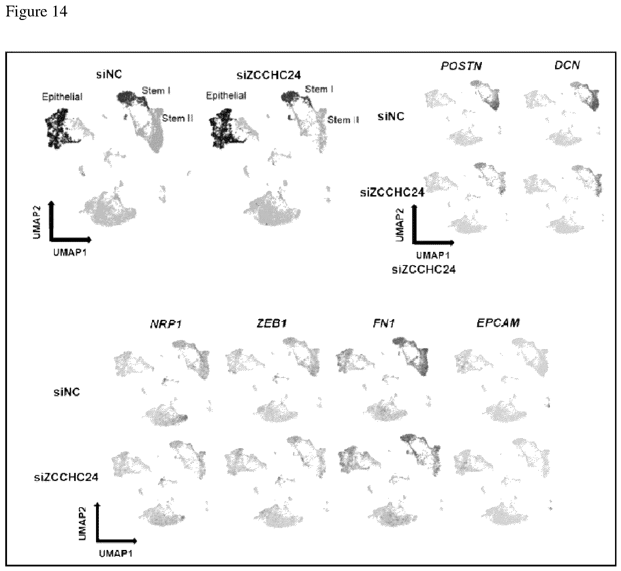

[Figure 14] Results of the single cell RNA-seq analysis of the PDX subcutaneous transplant model also confirmed that ZCCHC24 is specifically expressed in the NRP1 (+) NCAM1 (-) Stem II cell fraction. The same sample was sorted by FACS (Stem I cell fraction: NRP1 (+) NCAM1 (+), Stem II cell fraction: NRP1 (+) NCAM1 (-)).

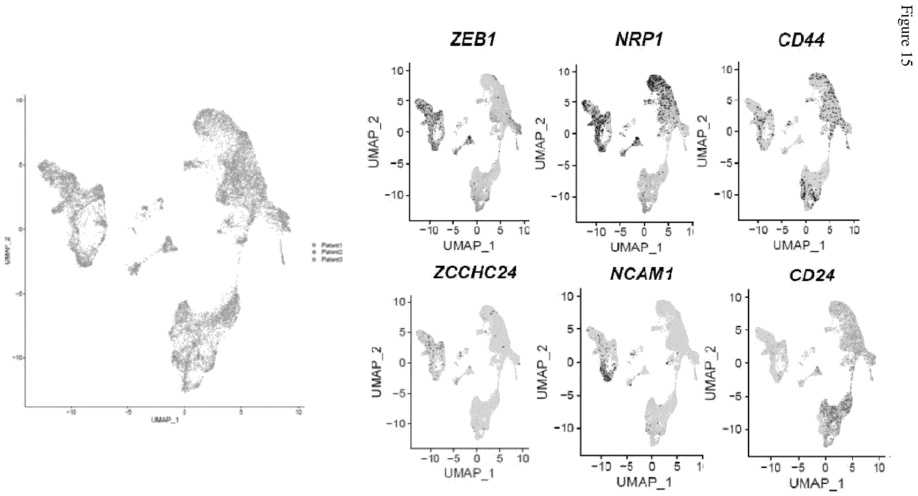

[Figure 15] As a result of re-transplanting the recovered Stem I cell fraction (NRP1 (+) NCAM1 (+)), Stem II cell fraction (NRP1 (+) NCAM1 (-)), and their mixture (Stem I + Stem II) into mice, the Stem II cell fraction showed higher tumorigenicity compared to the Stem I and Stem I+II cell fractions.

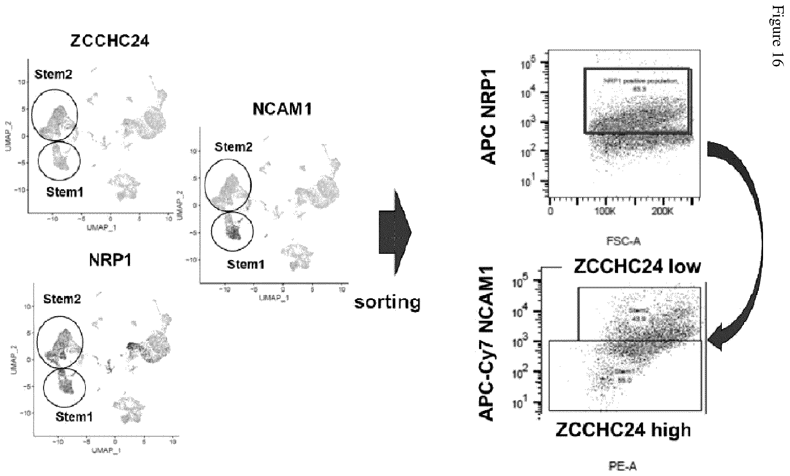

[Figure 16] As a result of reanalysis using scRNAseq of tumor samples formed by retransplantation of the Stem I, Stem II, and Stem I + Stem II cell fractions, it was found that they were fractionated into ECAM high (Stem I)/ECAM low (Stem II) and Epithelial, and the tumor sample had a cell fraction similar to that of the tumor before retransplantation.

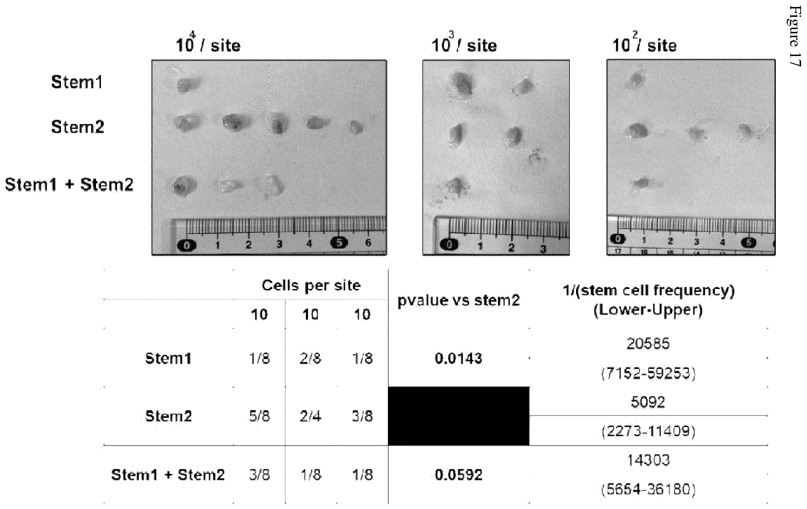

[Figure 17] As a result of compound screening using reporter cells labeled by inserting a HiBiT tag into the C terminus of ZCCHC24, 11 up-regulators (compounds with Z score > 3.0 or higher) and 9 down-regulators (compounds with Z score < -3.0 or lower) were identified (Figure 17A, Figure 17B). BET inhibitors and topoisomerase inhibitors were specifically extracted as up-regulators (Figure 17C), and microtubule polymerization inhibitors and Wee1/Chk1 inhibitors, which are important in the DNA damage response pathway, were identified as down-regulators. (Figure 17D).

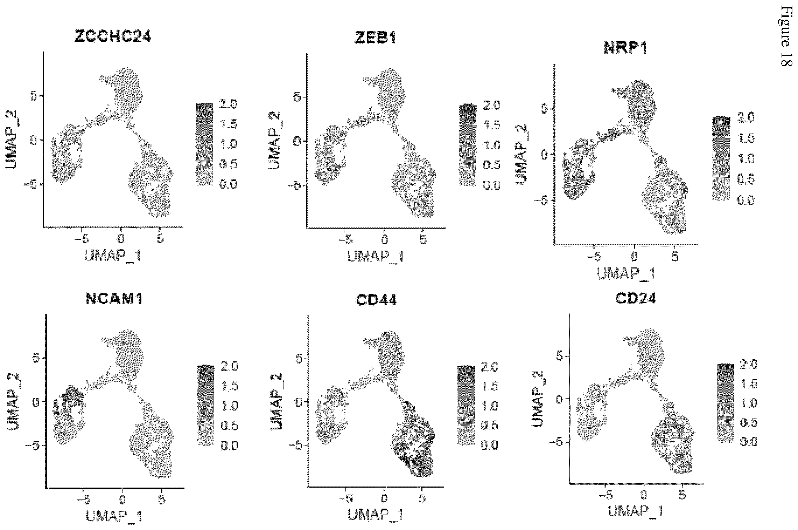

[Figure 18] The EC₅₀ concentration of the BET inhibitor JQ1 for the maximum increase in ZCCHC24 expression was approximately 50 nM, and it was revealed that it increases the expression of ZCCHC24 (Figure 18A). On the other hand, the IC₅₀ of JQ1 for the maximum cell survival inhibition in MDAMB231 was approximately 0.1 µM (Figure 18B). With respect to the transcription factor ZEB 1, the ChIP database of histone markers and previous ChIP-Seq data with ZEB1 revealed a region between the first and second exons where the H3K4Me1 and H3K27Ac histone markers show a peak and the peak of ZEB1 was also present in the region (Figure 18C). Furthermore, the expression of ZEB1 was dramatically increased by administration of JQ1 (Figure 18D). Furthermore, the JQ1-induced increase in the expression of ZCCHC24 was significantly reduced by knockdown of ZEB1 (Figure 18E).

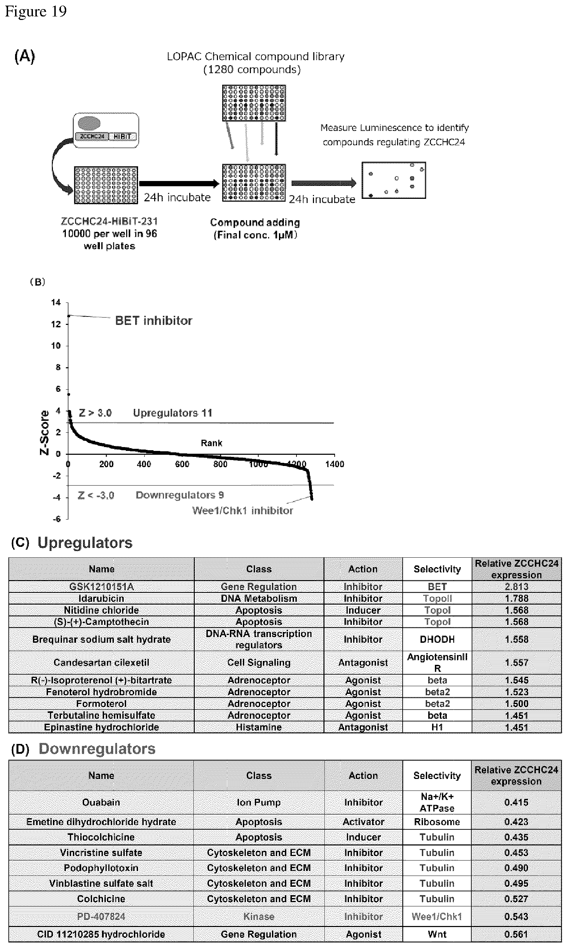

[Figure 19] The EC₅₀ concentration of the Wee1/Chk1 inhibitor PD407824 for maximally suppressing the expression of ZCCHC24 was approximately 50 nM (Figure 19A). On the other hand, although a certain degree of cell activity was suppressed by PD407824, the cell activity was not suppressed to the extent that IC₅₀ could be calculated (Figure 19B). Furthermore, when JQ1 and PD407824 were administered individually, no decrease in the cancer stem cell-like population was observed when JQ1 was administered, whereas there was a tendency for the cell population to decrease when PD407824 was administered and when JQ1 and PD407824 were administered together (Figure 19C, Figure 19D). Furthermore, in examination of the effect of suppressing cell proliferation by combining 100 nM JQ1 and 1 µM PD407824, it was confirmed that the combination had an additive effect (Figure 19E).

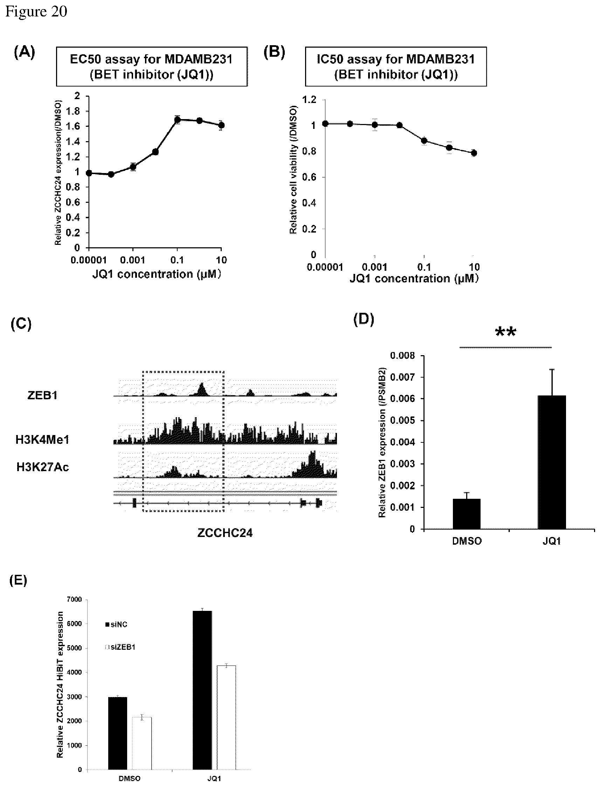

[Figure 20] When ZCCHC24 knockout mice were injected intraperitoneally with LPS, the period of survival after LPS injection was prolonged compared to wild type mice. It is suggested that ZCCHC24 is important as an RNA-binding protein that regulates inflammation.

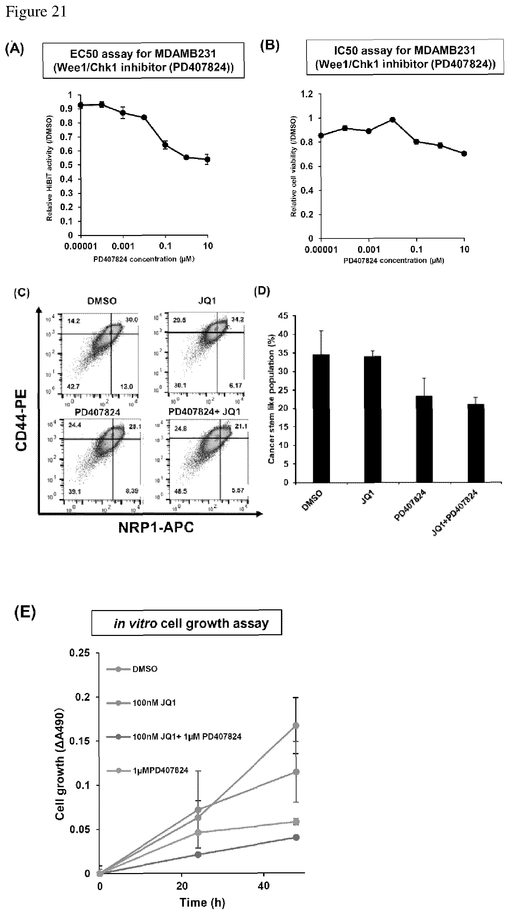

[Figure 21] When ZCCHC24 knockout mice were fed a high fat diet (HFD), weight gain was significantly suppressed compared to wild type mice. It was shown that ZCCHC24 may also possibly play an important role in obesity.

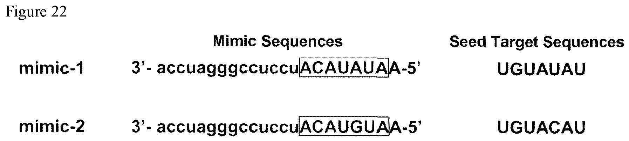

[Figure 22] The structure of miRNA (miR-PBE1 and miR-PBE2) having a sequence complementary to at least a part of the binding target sequence of the ZCCHC24 protein is shown.

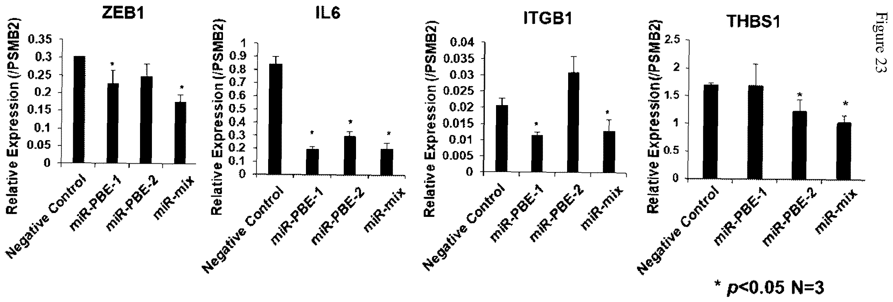

[Figure 23] When miR-PBE1 and/or miR-PBE2 were introduced into the MDAMB231 cell line, the expression of genes involved in breast cancer stemness and cancer cell invasion, such as ZEB1, IL-6, ITGB1, and THBS1, was suppressed.

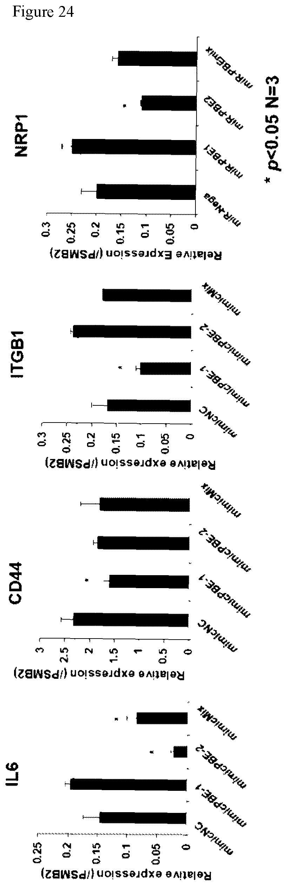

[Figure 24] When miR-PBE1 and/or miR-PBE2 were introduced into breast cancer TNBC patient-derived cells (PDX), the expression of genes which are involved in breast cancer stemness and cancer cell invasion, such as IL-6, CD44, ITGB1, and NRP1, was suppressed.

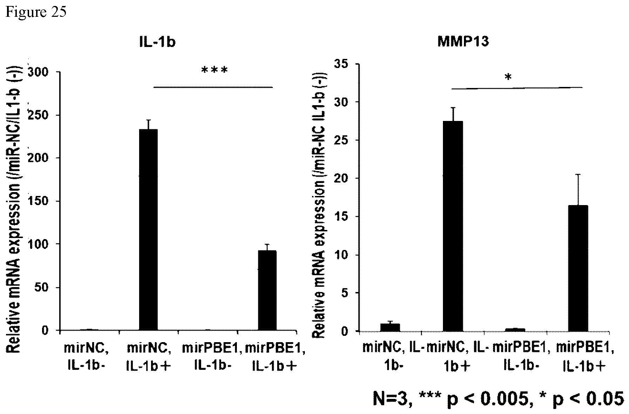

[Figure 25] When miR-PBE1 was introduced into the human chondrocyte cell line SW1353, the expression of MMP13 and IL1-β, which are important effector molecules in knee osteoarthritis, was suppressed.

### Mode for Carrying Out the Invention

As mentioned above, the present inventors have discovered that inhibitors of the ZCCHC24 protein or agents that suppress expression of the ZCCHC24 gene can be used as therapeutic drugs for TNBC, inflammatory disease, or obesity, and identified the Wee1/Chk1 inhibitor as a specific agent that suppresses the expression of the ZCCHC24 gene. The present invention will be explained in detail below.

### Triple negative breast cancer (TNBC)

Triple negative breast cancer (TNBC) is a breast cancer characterized by being estrogen receptor negative, progesterone receptor negative, and HER2 (human epidermal growth factor receptor 2) negative. TNBC accounts for approximately 20% of all breast cancers, and it has a very high recurrence rate within 3 years, and a shorter survival period after recurrence compared to other types of breast cancer. Further, hormone therapy and the molecular-targeted drug Herceptin therapy, which are commonly used to treat breast cancer, are ineffective, and the only drugs that can be expected to be effective are anticancer drugs. As a result, there are many cases that are difficult to treat, and many patients suffer from the side effects of anticancer drugs.

### Compositions for use in the treatment of TNBC

One embodiment of the present invention relates to compositions for use in the treatment and/or prevention of triple negative breast cancer, comprising as an active ingredient an inhibitor of the ZCCHC24 protein or an agent that suppresses the expression of the ZCCHC24 gene.

ZCCHC24 (Zinc Finger CCHC-Type Containing 24) is a protein with two different zinc finger domains. The present inventors have shown that, (1) ZCCHC24 is an RNA-binding protein that is strongly expressed in cancer tissues and cells, especially breast cancer stem cells; (2) ZCCHC24 promotes tumorigenicity by up-regulating the expression of a set of cancer-promoting proteins through post-transcriptional regulation; (3) cancer growth and tumorigenicity are suppressed by reducing the expression of ZCCHC 24; and (4) ZCCHC24 is a therapeutic target in refractory breast cancer such as triple negative breast cancer.

### <Agents that inhibit the function of the ZCCHC24 protein>

Therefore, for example, an agent that inhibits the function of the ZCCHC24 protein (hereinafter also referred to as a ZCCHC24 inhibitor or a ZCCHC24 protein inhibitor) can be used for the treatment and/or prevention of triple negative breast cancer. The ZCCHC24 inhibitor includes, for example, neutralizing antibodies, aptamers, miRNAs, antimiRs, antagomiRs, and other nucleic acid drugs and low molecular weight compounds, but is not limited thereto.

In the present specification, with respect to an agent that inhibits the function of the ZCCHC24 protein and an agent that suppresses the expression of the ZCCHC24 gene, the term "nucleic acid drug" includes nucleic acid molecules comprising any modified base. Modified nucleic acids include, but are not limited to, 2'-MOE, 2'-O-MCE, LNA (2'-4'BNA), ENA, AmNA, GuNA, scpBNA, 2'-OMe, phosphorothioate modification, and such.

### • Neutralizing antibodies

The neutralizing antibody may be a polyclonal antibody or a monoclonal antibody. Neutralizing antibodies can be produced by any method known to those skilled in the art. As the antigen, for example, the whole ZCCHC24 protein or a fragment thereof can be used. Antibody production may be outsourced to a contract manufacturer such as Eurofin Genomics Co., Ltd. (Tokyo). The mammal from which the antibody is derived is not particularly limited, and human antibodies, mouse antibodies, rat antibodies, rabbit antibodies, sheep antibodies, camel antibodies, or such can be used. When used for humans, the antibody may be a human antibody, a humanized antibody, or a chimeric antibody, but a human antibody is preferred. In addition, neutralizing antibodies may include antibody fragments. Thus, neutralizing antibodies can also include, for example, scFv, Fab, Fab', F(ab')₂, Fv, rIgG, nanobodies, peptibodies, minibodies, and diabodies. Further, the neutralizing antibody may comprise a ZCCHC24-binding peptide obtained by the phage display method or the like.

### • Aptamers

Aptamers are nucleic acid molecules (generally RNA) which form a three-dimensional structure that binds to a target. Aptamers may be used as the ZCCHC24 inhibitor. Aptamers that bind to ZCCHC24 can be obtained by any method known to those skilled in the art, for example, by the SELEX method.

### • Novel miRNAs

As the miRNA, novel miRNAs targeting the target gene of ZCCHCH24 can be used. An example of a miRNA for the present embodiment is described below.

MicroRNAs (miRNAs) present *in vivo* are short non-coding RNAs of 20-24 nucleotides that are involved in the post-transcriptional regulation of gene expression in multicellular organisms by affecting both mRNA stability and translation. In vivo, miRNAs are incorporated into RNA-induced silencing complexes (RISCs), and RISCs that incorporate miRNAs recognize their target mRNAs through incomplete base pairing with miRNAs, resulting in translational inhibition or destabilization of the target mRNA.

As shown in the examples below, the present inventors for the first time identified a novel target motif sequence "WGUWHWWA" (*1 in Table 1) as a target sequence for ZCCHC24 to bind to the 3' UTR of mRNA of a group of genes that characterize cancer stemness. The target motif sequence can be, for example, "UGUAHAWA" (*2 in Table 1), or "WGUWUWUA (i.e., (A/U)GU(A/U)U(A/U)U(A/U)UA)" (*3 in Table 1) (In the sequence, W represents A or U and H represents A or C or U.).

The novel miRNA of the present embodiment recognizes this target sequence of ZCCHC24 as a target. As a result of this, the miRNA of the present embodiment can act on the target gene of ZCCHC24 by competitively binding to ZCCHC24 to inhibit the function of the ZCCHC24 protein, that is, to suppress the expression-promoting effect of the target gene by ZCCHC24. At the same time, the miRNA of the present embodiment can cause inhibition of translation or destabilization of the mRNA of the target gene by the original miRNA function. Due to these dual effects, the miRNA of the present embodiment can exert an excellent effect as a ZCCHC24 inhibitor to suppress the expression of the target gene of ZCCHC24.

As an example, the miRNA in the present embodiment can be designed as an RNA molecule having a sequence consisting of:
(i) a seed sequence that recognizes the target sequence of ZCCHCH24;
(ii) an adenine or a uracil base on the 5' side of the seed sequence;
(iii) a scrambled sequence on the 3' side of the seed sequence.

The seed sequence can be configured to be complementary to at least a portion of the target sequence of ZCCHCH24, WGUWHWWA (*1 in Table 1) (also referred to herein as the "seed target sequence"). The base length of the seed sequence is generally 5 bases or more, preferably 6 bases or more, more preferably 7 bases or more. As an example, the base length of the seed sequence can be 5 to 7 bases.

The scrambled sequence can be any random sequence, but can be constructed so that the GC content of the full miRNA length is 40-60%, preferably 45-55%, and even more preferably 48-52%. Further, the scrambled sequence should also be configured so as not to cause complementary base pair formation with transcripts other than the target mRNA.

The miRNA can be a sequence with a full length of 19 to 25 bases, preferably 20 to 24 bases, more preferably 21 to 23 bases. Specific examples of miRNAs for the present embodiment are described in detail in the Examples. The adenine on the 5' side of the seed sequence may be replaced with uracil in the sequences described in the Examples below.

By configuring the miRNA as described above, miRNA single-stranding, RISC complex formation, mRNA recognition ability, and such can be improved, and its action as miRNA can be enhanced.

### • Low molecular compounds

A low-molecular compound may be used as the ZCCHC24 inhibitor. Small molecules that bind to ZCCHC24 can be designed by any method known to those skilled in the art, for example, by computer simulations based on the crystal structure of ZCCHC24.

### <Agents that suppress the expression of the ZCCHC24 gene>

Similar to the ZCCHC24 inhibitor, agents that inhibit the expression of the ZCCHC24 gene (hereinafter also referred to as the ZCCHC24 expression inhibitor), for example, can be used for the treatment and/or prevention of triple negative breast cancer. The ZCCHC24 expression inhibitor includes, but is not limited to, for example, low molecular compounds, peptides, proteins, antisense nucleic acids, siRNAs, miRNAs, antimiRs, antagomiRs, and other nucleic acid drugs.

Suppression of the expression of the ZCCHC24 gene can be carried out at any level, including suppression of mRNA transcription, degradation of transcribed mRNA, and inhibition of translation from mRNA to protein, without being limited thereto. Suppression of the ZCCHC24 gene expression can be evaluated by measuring the amount of the ZCCHC24 mRNA or protein using a known method.

Substances that can be used as the ZCCHC24 expression inhibitor can be identified, for example, by the screening method described in the present disclosure. Further, nucleic acid drugs such as antisense nucleic acids, siRNAs, miRNAs, antimiRs, antagomiRs, or such that can be used as the ZCCHC24 expression inhibitor can be designed based on sequences that are complementary to a portion of the ZCCHC24 gene sequence.

The miRNA of the present embodiment can be any miRNA that targets ZCCHC24, that is, any miRNA that causes inhibition of translation and/or destabilization of the ZCCHC24 mRNA.

The present inventors have identified the Wee1/Chk1 inhibitor as one of the compounds that reduces the expression of ZCCHC24. Furthermore, as used in the present disclosure, the term "Wee1/Chk1 inhibitor" refers to a substance that inhibits at least either of Wee1 and Chk1, but substances that inhibit both Wee1 and Chk1 are also effective.

As the Wee1 inhibitor, for example, PD407824, AZD-1775, ZN-c3, Debio-0123, IMP-7068, GDC-0575 (i.e., (R)-N-(4-(3-aminopiperidin-1-yl)-5-bromo-1H-pyrrolo[2,3-b]pyridin-3-yl) cyclopropanecarboxamide (CAS No. 1196541-47-5)), and MKK-1775 ((i.e., 1-[6-(2-hydroxypropan-2-yl)pyridin-2-yl]-6-[4-(4-methylpiperazin-1-yl)anilino]-2-prop-2-enylpyrazolo[3,4-d]pyrimidin-3-one (CAS No. 955365-80-7), also commonly known as adavosertib) may be used, but it is not limited thereto.

As the Chk1 inhibitor, for example, PD407824, ESP-01, PNT-737, BEBT-260, AZD7762, LY2603618, MK-8776, CHIR-124, PF-477736, rosovitin, SNS-032, dinaciclib, flavopiridol, AT7519, purvalanol A, RO-3306, SU9516, XL413, NU6027, P276-00, AZD5438, PHA-793887, JNJ-7706621, BMS-265246, milciclib, MK-8776, R547, or MKK-1775 ((i.e., 1-[6-(2-hydroxypropan-2-yl)pyridin-2-yl]-6-[4-(4-methylpiperazin-1-yl)anilino]-2-prop-2-enylpyrazolo[3,4-d]pyrimidin-3-one (CAS No. 955365-80-7), also commonly known as adavosertib) may be used, but it is not limited thereto. In one embodiment, milciclib and adavosertib are excluded from the Chk1 inhibitor.

WO2017/151554A1 illustrates the WEE1/CHK1 inhibitors as an example of DNA damage agents. WO2020/104777A1 illustrates the WEE1/CHK1 inhibitors as an example of DNA damage response inhibitors. These references are incorporated herein by reference in their entirety.

### BET inhibitors

BET (Bromodomain and extraterminal domain) proteins are proteins that recognize acetylated histones and regulate gene transcription through mobilization of transcription factors. Inhibition of the BET proteins has been shown to have antitumor activity.

The present inventors have found that the expression of ZCCHC24 is dramatically up-regulated by the action of BET inhibitors, which are expected to be novel therapeutic agents for triple negative breast cancer. This indicates that ZCCHC24 is involved in the mechanism by which resistance to treatment with BET inhibitors occurs. For example, the Wee1/Chk1 inhibitors are compounds that reduce the expression of ZCCHC24, and can be combined with existing BET inhibitors and other agents for the treatment of refractory breast cancer.

Thus, in some embodiments, compositions for use in the treatment and/or prevention of triple negative breast cancer that comprise as an active ingredient an inhibitor of the ZCCHC24 protein or an agent that inhibits expression of the ZCCHC24 gene as described above can be used in combination with a BET inhibitor. Also, in some embodiments, compositions for use in the treatment and/or prevention of triple negative breast cancer that comprise as an active ingredient an inhibitor of the ZCCHC24 protein or an agent that inhibits expression of the ZCCHC24 gene further comprises a BET inhibitor.

As the BET inhibitor, for example, JQ1, perabresib, BMS-986158, INCB-057643, ODM-207, PLX-2853, ABBV-744, BI-894999, BPI-23314, CC-90010, FT-1101, JAB-8263, mibebresib, SF -1126, or SYHA-1801 may be used, but it is not limited thereto.

### Compositions for use in the treatment of patients with the Stem II cell fraction

In addition, as mentioned above, the present inventors discovered a new breast cancer stem cell fraction, Stem II, in which ZCCHC24 shows specifically a high expression, and NRP1 (+ ) NCAM1(-) was identified as a combination of cell surface markers that can be used to identify this Stem II.

Thus, in some embodiments, the treatment subject of the compositions for use in the treatment and/or prevention of triple negative breast cancer in the present invention is a patient who has the stem cell fraction Stem II identified with NRP1(+) NCAM1(-). That is, in some embodiments, the present invention relates to compositions for use in the treatment of triple negative breast cancer, for use in the treatment of patients having the NRP1 (+) NCAM1 (-) cell fraction.

Further, in the present specification, "(+)" and "positive" have the same meaning with respect to the surface antigen, and mean that it is positive for the presence of the surface antigen. Similarly, "(-)" and "negative" mean negative for the presence of the surface antigen.

### Compositions for use in the treatment of inflammatory diseases

The findings and experimental results by the present inventors indicate that ZCCHC24 is a therapeutic target molecule in the treatment of a wide range of inflammatory diseases, without limitation to only triple negative breast cancer, and acts at the RNA level as an integrated regulator of inflammatory cytokines such as PDL1, IL-6, IL-8 and CXCL1, which are co-stimulatory inhibitory molecules.

Therefore, one embodiment of the present invention relates to compositions for use in the treatment and/or prevention of inflammatory diseases, which comprises as an active ingredient an inhibitor of the ZCCHC24 protein or an agent that suppresses the expression of the ZCCHC24 gene. The agent that suppresses the expression of the ZCCHC24 gene can be, for example, a Wee1/Chk1 inhibitor. In addition, the description of the present disclosure related to compositions for treatment of triple negative breast cancer applies similarly to compositions for use in the treatment and/or prevention of inflammatory diseases.

The inflammatory diseases include systemic inflammatory response syndrome (SIRS) including sepsis, rheumatoid arthritis (involvement of TNF and IL6), psoriatic arthritis (involvement of TNF, IL17, and IL12/23), psoriasis (involvement of TNF, IL17, and IL12/23), ankylosing myelitis (involvement of TNF and IL17), inflammatory bowel disease (involvement of TNF), SLE, atopic dermatitis (involvement of IL4/IL13), asthma (involvement of IL4/IL13 and IL5), COPD (involvement of IL5), gout (involvement of IL1), knee osteoarthritis (involvement of MMP13 and IL1-β), and such. Accordingly, the compositions of the present invention may also be used effectively in the treatment and/or prevention of these diseases.

### Compositions for use in the treatment of obesity

Inflammatory cytokines and chemokines such as IL-6, IL-8, CXCL1, and TNF*α* have been shown to play important roles in the pathogenesis of obesity in inflammatory cells such as macrophages that infiltrate the adipose tissue. The findings and experimental results by the present inventors show that ZCCHC24, as a molecule that broadly integrates and regulates these inflammatory cytokines and chemokines at the RNA level, is widely considered to be a therapeutic target molecule in the treatment of obesity.

Thus, one of the embodiments of the present invention relates to compositions for use in the treatment and/or prevention of obesity, comprising as an active ingredient an inhibitor of the ZCCHC24 protein or an agent that inhibits the expression of the ZCCHC24 gene. In addition, the description of the present disclosure related to compositions for the treatment of triple negative breast cancer is equally applicable to compositions for use in the treatment and/or prevention of obesity.

Furthermore, obesity is generally defined as "a state of excessive accumulation of fat in the adipose tissue with a body mass index (BMI = [weight (kg)]/[height (m)²]) of 25 or more". Obesity is mainly caused by an imbalance between energy intake and consumption (an increase in the ratio of intake to consumption) in physical activity and daily life. Thus, obesity encompasses, for example, food-induced insulin resistance and/or weight gain.

In the present specification, the treatment and/or prevention of obesity encompasses prevention of an increase in body weight and/or fat mass, as well as reduction of fat mass and/or body weight.

Furthermore, the composition for use in the treatment and/or prevention of obesity according to the present embodiment may also be effective in treating and/or preventing diseases, conditions, and complications caused by and/or related to obesity.

Diseases and conditions caused by and/or associated with overweight due to obesity include sleep apnea syndrome, orthopedic and musculoskeletal disorders, and the like.

Diseases, conditions, and complications resulting from and/or related to metabolic disorders caused by obesity include glucose intolerance/diabetes, dyslipidemia, fatty liver, hyperuricemia/gout, cardiovascular disease/cerebrovascular disease (atherosclerosis), nonalcoholic fatty liver disease, nonalcoholic steatohepatitis, obesity-related kidney disease, diabetic nephropathy, diabetic retinopathy, diabetic vasculopathy, diabetic neuropathy, hyperleptinemia , renal steatosis, pancreatic steatosis, cardiac steatosis, steatohepatitis, fibrosis, cirrhosis, chronic low-grade inflammation, hypertension, circulatory disease, and other inflammatory conditions and inflammatory diseases related to obesity.

### Pharmaceutical compositions

One embodiment of the present invention relates to pharmaceutical compositions for use in the treatment and/or prevention of triple negative breast cancer, inflammatory diseases, or obesity, comprising as an active ingredient an inhibitor of the ZCCHC24 protein or an agent that inhibits the expression of the ZCCHC24 gene. That is, one aspect of the present invention relates to the use of an inhibitor of the ZCCHC24 protein or an agent that inhibits the expression of the ZCCHC24 gene in the manufacture of a medicament for the treatment or prevention of triple negative breast cancer, inflammatory diseases, or obesity. Also, in some embodiments, a pharmaceutical composition for use in the treatment of triple negative breast cancer comprising as an active ingredient an inhibitor of ZCCHC24 protein or an agent that inhibits expression of the ZCCHC24 gene may further comprise a BET inhibitor.

The content ratio of the ZCCHC24 protein inhibitor or the agent that suppresses the expression of the ZCCHC24 gene in 100% by weight of the pharmaceutical composition can be appropriately set in the range of 0.001 to 99.99% by weight. Furthermore, the content ratio of the BET inhibitor can be appropriately set within the range of 0 to 99.99% by weight. Other components in the pharmaceutical composition according to the present invention are not particularly limited and can be appropriately selected according to the purpose, and include, for example, pharmaceutically acceptable carriers and additives. There are no particular limitations on the carrier or additive, and they can be selected appropriately depending on the dosage form or such, and may be any carrier, buffer, diluent, excipient, suspending agent, lubricant, adjuvant, vehicle, delivery system, emulsifier, disintegrant, absorbent, preservative, surfactant, coloring agent, flavor, or sweetener. The content in the pharmaceutical composition according to the present invention is not particularly limited either, and can be appropriately selected depending on the purpose. Thus, in some embodiments, a pharmaceutical composition can include or consist of an inhibitor of the ZCCHC24 protein or an agent that suppresses the expression of ZCCHC24 gene, and a carrier or buffer.

The dosage form of the pharmaceutical compositions in the present invention is not particularly limited and can be selected according to the desired method of administration, such as injectable form (solution, suspension, solid preparation for dissolution before use, and such), solid dosage form (tablet, capsule, suppository, powder, and such), and the like. As injections, for example, a pH adjuster, a buffering agent, a stabilizer, an isotonic agent, a local anesthetic, or such is added to the composition, and injections for subcutaneous, intramuscular, intravenous administration or such can be manufactured using conventional methods. Examples of a pH adjuster and the buffer include sodium citrate, sodium acetate, sodium phosphate and such. Examples of the stabilizer include sodium pyrosulfite, EDTA, thioglycolic acid, thiolactic acid and such. Examples of isotonic agents include sodium chloride, glucose and such. Examples of local anesthetics include procaine hydrochloride, lidocaine hydrochloride and such. The solid dosage form may be provided with an enteric coating.

There are no particular limitations on the method of administration of the pharmaceutical compositions of the present invention, and for example, either local or systemic administration can be selected depending on the dosage form of the pharmaceutical composition, the condition of the patient, and the like. Administration can be performed by, for example, intravenous administration, subcutaneous administration, intramuscular administration, oral administration, enteral administration, intravenous administration, tube feeding or such. Further, enteral administration is not limited to administration through the anus but also includes, for example, administration via a tube or the like inserted into the digestive tract from outside the individual, such as through a gastrostomy. The position of insertion is not limited to the intestine, but includes the esophagus, stomach, small intestine, large intestine, and such.

The subject of administration of the pharmaceutical composition according to the present invention is not particularly limited and can be selected according to the purpose, for example, humans, non-human mammals, such as mice, rats, cattle, pigs, monkeys, dogs, cats, or such, but preferably humans, especially human patients with triple negative breast cancer, inflammatory diseases, or human patients suffering from triple negative breast cancer, inflammatory diseases, or obesity. Further, the pharmaceutical compositions of the present invention may also be administered for the prevention of the development of triple negative breast cancer, inflammatory disease, or obesity, particularly for the prevention of recurrence. In some embodiments, the subject of administration may be a human having the stem cell fraction Stem II identified with NRP1(+) NCAM1(-).

The dosage of the pharmaceutical composition according to the present invention is not particularly limited and can be selected according to the form of administration, the age and weight of the subject of administration, the degree of desired effect, or such. The dosage of an inhibitor of the ZCCHC24 protein or an agent that inhibits the expression of the ZCCHC24 gene can be, for example, 100 to 1000000 nmol per day, and preferably 150 to 100000 nmol per day. The frequency of administration can be, for example, 1 to 100 times per month.

The timing of administration of the pharmaceutical composition according to the present invention is not particularly limited and can be appropriately selected depending on the purpose. For example, it may be administered prophylactically to patients susceptible to the above-mentioned diseases, or it may also be administered therapeutically to symptomatic patients. Moreover, there is no particular limitation on the number of administrations, and it can be appropriately selected depending on the age, body weight, degree of desired effect, or such of the subject to be administered.

### Treatment methods

One embodiment of the present invention is a method for treating triple negative breast cancer, inflammatory disease, or obesity in a subject in need of treatment and/or prevention, comprising an effective amount of an inhibitor of the ZCCHC24 protein or an agent that suppresses expression of the ZCCHC24 gene. The subject in need of treatment and/or prevention is a mammal, for example a human. The dosage can be determined as appropriate depending on the type of drug used and the subject to which it is administered. The route of administration can also be determined as appropriate depending on the type of drug used and the subject to which it is administered. The preferred route of administration includes, for example, intravenous administration.

Further, some embodiments also relate to methods of treating triple negative breast cancer, inflammatory disease, or obesity in a subject in need of treatment and/or prevention, comprising administering to the subject (i) an effective amount of an inhibitor of the ZCCHC24 protein or an agent that suppresses expression of the ZCCHC24 gene and (ii) an effective amount of a BET inhibitor. Herein, the administration of the inhibitor of the ZCCHC24 protein or the agent suppressing the expression of the ZCCHC24 gene and the administration of the BET inhibitor may be performed simultaneously or at different times. Further, the number of doses within a given period of time may also differ between the administration of the ZCCHC24 protein inhibitor or the agent that suppresses the expression of the ZCCHC24 gene and administration of the BET inhibitor.

### Test methods

One embodiment of the present invention relates to a method of testing for breast cancer. As mentioned above, the present inventors have found that ZCCHC24 is an RNA-binding protein that is strongly expressed in cancer tissues and cells, mainly breast cancer stem cells.

Thus, one embodiment of the present invention relates to a method of testing for breast cancer in a subject, comprising: a) measuring the amount of the ZCCHC24 protein in a body fluid sample originating from the subject; b) comparing the amount of the ZCCHC24 protein with a predetermined reference value, wherein a higher amount of the ZCCHC24 protein than the predetermined reference value indicates the possibility of breast cancer. That is, one aspect of the present invention relates to the use of the ZCCHC24 protein as a blood marker for breast cancer. Further, one aspect of the present invention may also be said to relate to a method of testing for breast cancer in vitro, a method of measuring a blood marker for breast cancer, or a method of obtaining an indicator of the likelihood of breast cancer.

Measurement of the amount of the ZCCHC24 protein can be performed, for example, by ELISA in vitro, but it is not limited thereto. The fluid sample can be, for example, isolated blood, e.g., plasma or serum samples.

The predetermined reference value for comparison with the amount of the ZCCHC24 protein in a blood sample derived from the test subject can be determined, for example, based on the average amount of the ZCCHC24 protein in body fluid samples derived from a plurality of healthy individuals.

The method for measuring the amount of the ZCCHC24 protein is not particularly limited, but includes, for example, immunostaining.

In some embodiments, the breast cancer to be tested may be specifically triple negative breast cancer.

In addition, as described above, the present inventors also discovered a new breast cancer stem cell fraction, Stem II, which specifically shows high expression of ZCCHC24, and identified NRP1(+) NCAM1(-) as a combination of surface antibodies that can identify this Stem II.

Thus, one embodiment of the present invention relates to a method of testing for breast cancer in a test subject, comprising determining whether a cell sample derived from the test subject has a cell fraction of NRP1(+) NCAM1(-), wherein the presence of the cell fraction indicates the possibility of breast cancer. That is, one aspect of the present invention relates to the use of a combination of the cell surface markers NRP1(+) and NCAM1(-) as a test marker for breast cancer.

Further, one embodiment of the present invention relates to a method for testing the state of breast cancer in a test subject, wherein the testmethod comprises determining whether or not a cell sample derived from the test subject has a cell fraction of NRP1(+) NCAM1(-), and judging the breast cancer to be of high malignancy if the subcellular fraction is present. In other words, one aspect of the present invention relates to the use of a combination of the cell surface markers NRP1(+) and NCAM1(-) as an indicator of breast cancer malignancy.

Further, one embodiment of the present invention relates to a method for testing the state of breast cancer in a test subject, comprising determining whether a cell sample derived from the test subject has a cell fraction of NRP I (+) NCAM1(-), and if the cell fraction is present, identifying the subject as being eligible for administration of a composition for use in the treatment of triple negative breast cancer. In some embodiments, the composition for use in the treatment of triple negative breast cancer is a composition for use in the treatment of triple negative breast cancer, comprising as an active ingredient an inhibitor of the ZCCHC24 protein or an agent that suppresses the expression of the ZCCHC24 gene.

The cell sample can be a tumor-like site obtained from the subject.

Methods for determining whether a cell sample has a NRP1(+) NCAM1(-) cell fraction include, for example, single cell RNA sequence analysis (scRNAseq), fluorescence-activated cell sorting (FACS), immunostaining, and such.

Further, one aspect of the present invention can also be said to relate to a method of testing for breast cancer or state of breast cancer in vitro, a method of measuring test markers for breast cancer, or a method of obtaining an indicator of the likelihood or degree of malignancy of breast cancer.

### Screening methods

One embodiment of the present invention relates to a method of screening for agents for use in the treatment of triple negative breast cancer. In some embodiments, the screening method comprises the steps of (i) preparing cells expressing ZCCHC24, (ii) contacting the cells with a test substance, (iii) measuring the expression level of ZCCHC24, and (iv) selecting a substance that reduces the expression level of ZCCHC24. Further, one embodiment of the present invention also relates to a method of screening for agents for use in the treatment of an inflammatory diseases. In some embodiments, the screening method comprises the steps of (i) preparing cells expressing ZCCHC24, (ii) contacting the cells with a test substance, (iii) measuring the expression level of ZCCHC24, and (iv) selecting a substance that reduces the expression level of ZCCHC24. Further, one embodiment of the present invention also relates to a method of screening for agents for use in the treatment of obesity. In some embodiments, the screening method comprises the steps of (i) preparing cells expressing ZCCHC24, (ii) contacting the cells with a test substance, (iii) measuring the expression level of ZCCHC24, and (iv) selecting a substance that reduces the expression level of ZCCHC24.

In some embodiments, the ZCCHC24-expressing cells may be cells modified to express labeled ZCCHC24. The label for use can be any label known to those skilled in the art, for example, a fluorescent protein or such. The cells for use can be, for example, MDAMB231 cells.

The test substance can be, for example, a small molecule, peptide, protein, or biologically derived extract contained in a compound library. Contact between the cells and a test substance can be made, for example, by adding the test substance to the cell culture medium. The expression level of ZCCHC24 can be measured, for example, by measuring the amount of the ZCCHC24 mRNA or the amount of the ZCCHC24 protein. These measurement methods can be performed by any method known to those skilled in the art, for example, PCR, ELISA, Northern blotting, Southern blotting, or such. The decrease in the expression of ZCCHC24 can be made by comparison with a predetermined reference value. For example, the expression level of ZCCHC24 measured under the same conditions except for the presence or absence of the test substance can be used as the reference value.

Substances identified by such screening methods may be used to treat triple negative breast cancer, inflammatory diseases, or obesity.

The following examples are provided to illustrate the present invention in detail, but the present invention is not limited in any way by these examples.

### Examples

### Materials and methods

### Single-cell RNA-Seq reanalysis

Reanalysis of single-cell RNA seq data for five human breast cancer specimens from a previous report (Wu et al, EMBO J. 2020) was performed using the Cell ranger (10x genomics) and Seurat (https://satijalab.org/seurat/articles/ get_started.html) software.

### RNA-Seq

2 × 10⁵ cells of the human triple negative breast cancer cell line MDAMB231 were seeded in 6-well plates. Twenty-four hours later, a negative control (Stealth RNAi (trademark) siRNA Negative Control Hi GC (*4 in Table 1), Thermofisher Scientific) or siRNA against ZCCHC24 (HSS137253, stealth RNA (*5 in Table 1), Thermofisher Scientific) were transfected using RNA iMax (Thermofisher Scientific). After another 48 hours, RNA was harvested using the Relia RNA miniprep system (Promega). A library for RNA-Seq was prepared using 500 ng of RNA using NEB Next rRNA depletion kit v2 (NEB) and NEBNext Ultra II RNA library prep kit for Illumina (NEB). RNA-Seq was performed using Next-Seq (Illumina). Adapter processing for RNA-Seq was performed using Trim Galore (https://www.bioinformatics.babraham.ac.uk/projects/trim_galore/). Mapping was performed using STAR (https://github.com/alexdobin/STAR). Quantification was performed using RSEM (R package). The expression changes of genes were determined using iDEP 91 (http://bioinformatics.sdstate.edu/idep/).

### qPCR analysis

2 × 10⁵ human cells derived from breast cancer patients (PDX) were seeded in 6-well plates. Twenty-four hours later, control or siRNA against ZCCHC24 (*4 and 5 in Table 1) was transfected. Forty-eight hours later, RNA was harvested using the Relia RNA miniprep system (Promega). Reverse transcription was performed using dNTPs (Toyobo), random primers (Toyobo), and Prime Script (Takara). Then qPCR was performed using primer sets for CD44, ZCCHC24, ZEB1, and NRP1 (*6-9 in Table 1 (Sequence ID NOs: 1-8)).

### eCLIP-Seq

MDAMB231 cells in which ZCCHC24 was overexpressed with doxycycline were collected, and subjected to 254 nm UV-crosslinking at 300 mJ/cm² on ice with an Ultraviolet Crosslinker (UVP, CA, USA). Cells were collected, centrifuged, and resuspended in lysis buffer (50 mM Tris-HCl pH 7.4, 100 mM NaCl, 1% NP-40 (Igepal CA630), 0.1% SDS, 0.5% sodium deoxycholate, 1:100 protease inhibitor). After incubation on ice for 15 minutes, the samples were sonicated in a Bioraptor (CosmoBio, Tokyo, Japan) at 4°C, 30 sec on/30 sec off setting for 5 min, and then processed in a thermomixer at 1200 rpm, 37°C for 5 minutes, with 10 µl of 1:100 diluted RNase I (Thermo Fisher Scientific, MA, USA) and 2 µl of Turbo DNase (Thermo Fisher Scientific) per sample. After incubation, 11 µl of a mouse RNase inhibitor was added immediately and centrifuged at 4°C for 15 min. For immunoprecipitation, a mouse anti-FLAG antibody (MBL) was coupled to Dynabeads Protein G (Thermo Fisher Scientific) according to the manufacturer's protocol, and the coupled antibody was washed with lysis buffer. The entire lysate and coupled antibody were mixed and rotated overnight at 4°C. The antibody-bound proteins and RNA-protein complexes were separated on a magnetic stand and washed with a wash buffer (20 mM Tris-HCl, pH 7.4, 10 mM MgCl₂, 0.2% Tween-20), a high salt wash buffer (50 mM Tris-HCl pH 7.4, 1 M NaCl, 1 mM EDTA, 1% NP-40, 0.1% SDS, 0.5% sodium deoxycholate), and FastAP buffer (10 mM Tris HCl pH7.4, 5 mM MgCl₂, 100 mM KCl, 0.02% Triton X-100). The bound RNA was treated with FastAP alkaline phosphatase (Thermo Fisher Scientific) for 30 min and T4 polynucleotide kinase (PNK) (NEB) for 45 min. Then, the bound beads were then washed with a cold wash buffer, a high salt wash buffer, and a ligase buffer (50 mM Tris-HCl pH 7.5, 10 mM MgCl₂). The bound RNA was ligated for 3 hours with a 3' RNA linker using high-concentration RNA ligase (NEB) and RNA adapters (*11 (Sequence ID NO: 11) in Table 1), followed by washing in a cold wash buffer and a high salt concentration wash buffer. The RNA-protein complexes were extracted with the NuPAGE sample buffer (Invitrogen), and SDS-PAGE was performed on them. They were then transferred to nitrocellulose membranes. Proteinase K (NEB), acid phenol/chloroform/isoamyl alcohol (Nippon Gene, Tokyo, Japan), and the Quick-RNA miniprep kit (Zymo Research, CA, USA) were used to cut out target regions of the membrane and extract RNA from the membrane. The purified RNA was reverse transcribed with the reverse transcription primer (*12 (Sequence ID NO: 12) in Table 1) and TGIRT-III enzyme (InGex, MO, USA). After treatment with ExoSAP-IT (Thermo Fisher Scientific), the rand103Tr3 linker (*13 (Sequence ID NO: 13) in Table 1) was ligated to the 5' region of the cDNA using high concentration RNA ligase (NEB) and allowed to stand overnight at room temperature. The adapter-ligated cDNA was amplified by PCR using the Q5 PCR enzyme (NEB) for 15 cycles, purified with the Ampure XP beads (Beckman Coulter, CA, USA) and gel purified. The CLIP-Seq library was sequenced on a Next-Seq 500 (Ilumina).

Adaptor sequences were removed from the sequence data by Cut-adapt. Reads with adaptors removed were mapped against the GRCh38 genomic data using STAR, and duplicate reads were removed using the UMI tool.

### PAR- CLIP

The human triple negative breast cancer cell line, MDAMB231 cell line, expressing ZCCHC24 was treated with 100 µ M 4SU (Sigma-Aldrich) for 24 hours. The cells were UV cross-linked using a UV crosslinker (UVP) at 365 nm and 500 mJ/cm². The cells were collected and placed on ice for 15 min with lysis buffer (50 mM Tris-HCl (pH 7.4), 100 mM NaCl, 1% NP-40 (Igepal CA630), 0.1% SDS, 0.5% sodium deoxycholate, protease inhibitor (1: (100)). Then, the samples were crushed on and off repeatedly using a Bioraptor (CosmoBio) in low settings at 4°C for 5 minutes. The RNA in the samples was then fragmented using 10 µL of RNase I (1: 100; Thermo Fisher Scientific) and 2 µL of Turbo DNase (Thermo Fisher Scientific) at 37°C, 1200 rpm for 5 minutes. After that, 11 µL of a mouse RNase inhibitor (Thermo Fisher Scientific) was added and it was centrifuged at 4°C for 15 min. An anti-FLAG antibody (MBL, Fla-1) was linked with Dynabeads Protein G ((Thermo Fisher Scientific) at a ratio of 1:1000 and immunoprecipitated at 4°C overnight. The antibody-bound proteins and RNA-protein complexes were purified using a magnetic stand (Invitrogen), and washed using a wash buffer (20 mM Tris-HCl (pH 7.4), 10 mM MgCl₂, 0.2% Tween-20), a high-salt wash buffer (50 mM Tris-HCl (pH 7.4), 1 M NaCl, 1 mM ethylenediaminetetraacetic acid, 1% NP-40, 0.1% SDS, and 0.5% sodium deoxycholate), and FastAP buffer (10 mM Tris-HCl (pH 7.4), 5 mM MgCl₂, 100 mM KCl, and 0.02% Triton X-100). The bound RNA was treated with FastAP alkaline phosphatase (Thermo Fisher Scientific) for 30 min and T4 polynucleotide kinase (PNK) (NEB) for 45 min. The bound RNA was then linked to an adapter on the 3' side for 3 hours, using a high concentration of RNA ligase (NEB) and an RNA adapter (*11 (Sequence ID NO: 11) in Table 1). After that, the RNA was washed in a wash buffer and a high salt wash buffer. The RNA-protein complexes were extracted using the NuPAGE sample buffer (Invitrogen) and subjected to SDS-PAGE. Then, the degraded samples were transferred to nitrocellulose membranes (0.2 µm). Regions with the target proteins on the blot were excised and RNA was extracted using proteinase K (NEB), acid phenol/chloroform/isoamyl alcohol (Nippon Gene), and the Quick-RNA miniprep kit (Zymo Research). Purified RNA was reverse transcribed using the TGIRT-III enzyme (InGex) together with reverse transcription primers (*12 (Sequence ID NO: 12) in Table 1). The cDNA was incubated with ExoSAP-IT (Thermo Fisher Scientific). In addition, the 5' end of the cDNA was ligated with the rand103Tr3 linker (*13 (Sequence ID NO: 13) in Table 1) overnight at room temperature using high concentration RNA ligase (NEB). The adapter-ligated cDNA was amplified by 15 cycles of PCR using the Q5 PCR enzyme (NEB) and purified with the Ampure XP beads (Beckman Coulter). The samples were then recovered by gel purification. The CLIP-Seq library was sequenced using Next-Seq 500 (Illumina).

The analysis of PAR-CLIP is as follows. Adaptor sequences in sequencing reads were removed using Cutadapt (https://cutadapt.readthedocs.io/en/stable/guide.html). STAR (https://github.com/alexdobin/ STAR) was used to map reads to the GRCh38 genome, and duplicate reads were removed using UMI Tools (https://umi-tools.readthedocs.io/en/latest/reference/extract.html). Annotation of the read data was performed using RSeqC (http://rseqc.sourceforge.net/).

### BRIC-Seq

1 × 10⁶ MDAMB231 cells were seeded in 15 cm dishes. Twenty-four hours later, control or ZCCHC24 siRNA (*4, 5 in Table 1) was transfected using RNA iMax (Invitrogen). Twenty-four hours later, 150 µ M (final concentration) of BrdU (Sigma Aldrich) was added. After two washes with PBS, harvesting was performed at 0 and 1 hour using TRIZOL (Thermofisher Scientific). To measure the degradation rate of RNA, immunoprecipitation with an anti-BrdU antibody (MBL, 2B1) was performed as follows. The 45 µg of RNA recovered was immunoprecipitated with 5 µg of the anti-BrdU antibody (MBL, 2B1) and IP buffer (1% Triton in PBS) and incubated at 4°C for 2 hours with rotation. After washing, the RNA was recovered using NEB's Monarch RNA cleanup kit (NEB). The library of recovered RNA was prepared for NGS using the NEBNext Ultra II RNA library prep kit for Illumina (NEB). The library was subjected to RNA-Seq by HiSeq (Illumina). Then, the adapter was removed from the sequence data using Cutadapt (https://cutadapt.readthedocs.io/en/stable/guide.html). The reads were mapped against the GRCh38 genome using STAR (https://github.com/alexdobin/STAR). BridgeR (R package) was used to measure the RNA degradation rates.

### Actinomycin D test

MDAMB231 or HCC38 were plated in 24 well plates at a rate of 5 × 10⁴/well. Twenty-four hours later, 20 µ M of siRNA (Thermofisher Scientific) was transfected together with 0.5 *µ* L or 3 *µ* L of RNA iMax (Thermofisher Scientific). Forty-eight hours post-transfection, 10 µM of ActinomycinD was added to the medium and RNA was collected at 0, 1, and 2 hours. RNA collected by the Relia Prep RNA mini prep (Promega) was reverse transcribed with Random Primer (Toyobo), dNTP (NEB), and Prime Script (Takara), and the expression changes of target genes were evaluated by qPCR.

### Sphere formation assay

2 × 10⁵ cells each of the human breast cancer cell lines MDAMB231 and HCC38 were seeded in 6-well plates. After 24 hours, the control or siRNA against ZCCHC24 (*4, 5 in Table 1) was transfected. After 24 hours, the cells were cultured for 1 week in DMEM F-12 medium (Gibco) supplemented with 20 ng/mL EGF (R&D), 20 ng/mL FGF (Wako), and B27 supplement; and 10 field images were randomly taken and the number of Spheres was calculated.

### Extreme dilution assay (ELDA)

2 × 10⁵ cells each of the human breast cancer cell lines MDAMB231 and HCC38 were seeded in 6-well plates. Twenty-four hours later, the control or siRNA against ZCCHC24 (*4, 5 in Table 1) was transfected. After 24 hours, 250, 125, 62.5, 31, and 15 cells were seeded in each well (N = 8), and the cells were cultured for 1 week in DMEM F-12 medium (Gibco) supplemented with 20 ng/mL EGF (R&D), 20 ng/mL FGF (Wako), and B27 supplement; and the presence of Spheres was measured.

### In vivo colony formation assay

Twenty-four hours after transfection with the control or siRNA against ZCCHC24 (*4, 5 in Table 1), subcutaneous transplantation of the human breast cancer cell line MDAMB231 or breast cancer PDX into female nude mice (NOG mice (Oriental Yeast)) was performed at a rate of 1 × 10⁴, 10³, 10² cells/site at 7 weeks of age together with 50% Matrigel (Corning). The number of tumors formed was counted after 1 month.

### Single cell RNA-Seq analysis of the PDX xenograft model

Negative control or the ZCCHC24 siRNA (*4, 5 in Table 1) (Thermofisher Scientific) was transfected into PDX. After 24 hours, 10³ siRNA-transfected cells were subcutaneously transplanted into 7-week-old female NOG mice (Oriental Yeast) with 50% Matrigel (Corning). After 19 days, the grown tumors were collected and treated with 3 µg/ml of collagenase (Wako). A library was prepared from the treated tumors using the Chromium Next GEM Chip G Single Cell Kit (10x chromium). The prepared library was sequenced using Nova-Seq (Illumina). Sequence data were analyzed using Seurat (https://satijalab.org/seurat/articles/get_started.html), and breast cancer stem cell fractions were analyzed.

### scRNAseq analysis of the NOG mouse subcutaneous transplantation model of patient-derived breast cancer specimens

Tumor specimens from three triple negative breast cancer patients were subcutaneously transplanted into NOG mice. When the tumor reached an appropriate size, it was removed and treated with collagenase (Wako) to break down the tumor mass. A library was prepared from the treated tumors using the Chromium Next GEM Chip G Single Cell Kit (10x chromium). The prepared library was sequenced using Nova-Seq (Illumina). Sequence data were analyzed using Seurat (https://satijalab.org/seurat/articles/get_started.html), and breast cancer stem cell fractions were analyzed.

### Single cell RNA-Seq analysis of the retransplantation model

PDX was subcutaneously transplanted into mice together with Matrigel (Corning #354262) at a ratio of 10³ cells/site. One month later, the formed tumors were removed and treated with collagenase. Sorting by FACS was performed using antibodies against surface antigens NRP1 and NCAM1 (NRP1-APC antibody: R&D, FAB3870A, NCAM1-APC-Cy7 antibody: BioLegend BL318331). The Stem I cell fraction was sorted based on NRP1 (+) NCAM1 (+), and Stem II was sorted based on NRP1 (+) NCAM1 (-).

Regarding the sorted cells, cells of the Stem I cell fraction, cells of the Stem II cell fraction, and mixed cells of the Stem I cell fraction and Stem II cell fraction were subcutaneously transplanted with Matrigel (Corning, #354262) into 7-week-old female NOG mice (Clea Japan) at a ratio of 10⁴, 10³, or 10² cells/site, and the tumors were sampled 6 weeks after transplantation. In addition to evaluation of the ability to form tumor, a scRNAseq analysis was performed on samples in which Stem II was retransplanted at 10² cells/site using 10x chromium (10x genomics) to generate an NGS library. The prepared NGS library was sequenced using NovaSeq (Ilumina), and the sequenced reads were analyzed using mapping by cell ranger (https://support.10xgenomics.com/single-cell-gene-expression/software/pipelines/latest/what-is-cell-ranger) and Seurat (https ://satij alab.org/seurat/).

### Compound screening using HiBiT tag knock-in cells

HiBiT tagged-MDAMB231 cells with were produced using the procedure below. First, crRNA (*14 (SEQ ID NO: 14) in Table 1) (IDT) was resuspended in the Nuclease-Free Duplex buffer (IDT) to a final concentration of 100 µM, and incubated at 95°C for 5 minutes together with the same amount of tracrRNA (IDT). The oligo complex was then slowly cooled down to room temperature and incubated with ALT-R Cas9 Nuclease V3 (61 µM) (Integrated DNA Technologies, IA, USA) for 20 minutes at room temperature to form the Cas9 complex. Next, a single-stranded DNA oligo (*15 (SEQ ID NO: 15) in Table 1) (comprising the HiBiT sequence and a sequence complementary to the sequence of the C-terminal region of ZCCHC24) and Cas9 complex were co-transfected into MDAMB231 cells by an electroporation method using the NEPA21 super electroporator (NEPAGENE). After single cell cloning, cells inserted with the HiBiT tag were collected.

The compound screening procedure is as follows. HiBiT tag-inserted cells were plated at 1 × 10⁴ cells in a 96-well plate, cultured for 24 hours, and then reacted with a low molecular compound library (LOPAC; Sigma Aldrich) at 1 µM (final concentration) for 24 hours. After the culture medium was discarded, cells were incubated with 12.5 µL PBS, 12.5 µL lysis buffer (Promega), 0.5 µL substrate (Promega), and 0.25 µL LgBiT (Promega) for 10 minutes at room temperature in the dark. The luminescence intensity was measured using ARVO X3 (Perkin Elmer).

### EC50 (Effective concentration 50) assay

1 × 10⁴ HiBiT tag-inserted cells were seeded in a 96-well plate. After 24 hours, 0.1% DMSO or 10⁻⁵, 10⁴, 10⁻³, 10⁻², 10⁻¹, 1, 10 µM of JQ1 (Selleck) or PD407824 (Sigma Aldrich) was added. After 24 hours, the medium was discarded, and 12.5 µL PBS, 12.5 µL Lytic Buffer from the HiBiT kit (Promega), 0.5 µL substrate, and 0.25 µL LgBiT were added. After 10 minutes of reaction at room temperature, luminescence was measured using ARVO X3 (Perkin Elmer).

### IC50 (Inhibitory concentration 50) assay

2 × 10³ HiBiT tag-inserted cells were seeded in a 96-well plate. After 24 hours, 0.1% DMSO or 10⁻⁵, 10⁻⁴, 10⁻³, 10², 10⁻¹, 1, 10 µM of JQ1 (Selleck) or PD407824 (Sigma Aldrich) was added. After 48 hours, 20 µL of Cell Titer Glo (Promega) was added. After 30 minutes of reaction at 37°C, the absorbance of A490 was measured using ARVO X3 (Perkin Elmer).

### Reanalysis of ChIP-Seq data

The ChIP-Seq data of ZEB1, H3K2Ac, and H3K4Me1 from Rhie et al, BMC Genomics (2014) and Katsura et al, Mol Oncol. (2017) were reanalyzed.

Rhie SK, Hazelett DJ, Coetzee SG, Yan C, Noushmehr H, Coetzee GA. Nucleosome positioning and histone modifications define relationships between regulatory elements and nearby gene expression in breast epithelial cells. BMC Genomics. 2014; 15:331

Katsura A, Tamura Y, Hokari S, Harada M, Morikawa M, Sakurai T, et al. ZEB1-regulated inflammatory phenotype in breast cancer cells. Mol Oncol. 2017; 11(9): 1241-62

### qPCR analysis on JQ1-treated MDAMB231

2 × 10⁵ MDAMB231 cells were seeded in a 6-well plate. After 24 hours, 0.1% DMSO or 100 nM of JQ1 was added. After 24 hours, RNA was extracted using the Relia Prep RNA MiniPrep (Promega) and reverse transcribed using Random Primer (Takara), dNTP (NEB), and Prime Script (Takara). For this cDNA, the quantification was carried out by qPCR of PSMB2 (*10 (SEQ ID NOs: 9, 10) in Table 1) and ZCCHC24 (*6 (SEQ ID NOs: 1, 2) in Table 1).

### Evaluation of the effect of ZEB1 siRNA knockdown by JQ1 treatment

1 × 10⁴ HiBiT tag-inserted cells were seeded in a 96-well plate. After 24 hours, transfection of 0.5 µL of siRNA for control and ZCCHC24 was performed together with 3 µL of RNA iMax (Thermofisher). Twenty-four hours later, 0.1% DMSO or 100 nM of JQ1 was added. After 24 hours, 12.5 µL PBS, 12.5 µL Lytic Buffer from the HiBiT kit (Promega), 0.5 µL of Substrate, and 0.25 µL of LgBiT were added. After 10 minutes of reaction at room temperature, luminescence was measured using ARVO X3 (Perkin Elmer).

### Analysis of breast cancer stem cell percentage after drug treatment by FACS

2 × 10⁵ MDAMB231 cells were seeded in a 6-well plate. After 24 hours, 0.1% DMSO, 100 nM of JQ1, 1 µM of PD407824, or both were added. Twenty-four hours later, cells were collected, stained with CD44-PE (IM7, Biolegend) and NRP1-APC (FAB3870A, R&D), and subjected to FACS analysis using FACS Calibur (BD).

### Cell growth assay

2 × 10³ MDAMB231 cells were seeded in a 96-well plate. After 24 hours, 100 nM JQ1 (Selleck) or 1 µM PD407824 (Sigma Aldrich) was added. Then, at 0, 24, and 48 hours later, the number of cells was quantified by absorbance measurement using Cell Titer Glo (Promega) and ARVO X3 (Perkin Elmer).

### Generation of the ZCCHC24 KO mice

KO mice were generated using the CRISPR-Cas9 system, by introducing the synthesized gRNA (*16 (SEQ ID NO: 16) in Table 1) together with Cas9 mRNA into the fertilized eggs of BDF1 mice.

### LPS shock model

ZCCHC24KO mice and wild type mice as control were intraperitoneally injected with LPS (lipopolysaccharide) at a ratio of 15 mg/kg. Thereafter, the survival of the mice was confirmed every 12 hours, and a survival curve was drawn.

### High fat diet model

A high fat diet was continuously fed to ZCCHC24KO mice and wild type mice as control. Body weight was also measured and recorded weekly.

### Novel nucleic acid drug experiment (miR-PBE1, 2)

Mimic-miR-RNA that binds complementarily to the RNA sequence ("UGUAHAWA" (*2 in Table 1)) which ZCCHC24 binds to was designed. As the seed target sequence, "UGAUAUAU" (*17 in Table 1) or "UGUACAU" (*18 in Table 1) was used.

The sequences are miR-PBE1 (5'-AAUAUACAUCCUCCGGGAUCCA-3') (*19 (SEQ ID NO: 17) in Table 1), miR-PBE2 (5'-AAUGUACAUCCUCCGGGAUCCA-3') (*20 (SEQ ID NO: 18) in Table 1) (Thermofisher miRNA mimics). These were introduced into the TNBC cell line MDAMB231, TNBC patient-derived cells (PDX), and the human chondrocyte cell line SW1353 together with the Lipofectamine RNA iMax (Invitrogen) to a final concentration of 25 nM or 50 nM. The samples were collected after 48 hours. SW1353 was stimulated with 10 nM human IL-1β for 6 hours before harvesting. After sample collection, RNA was purified using the Relia-Prep RNA Miniprep Systems (Promega) and reverse transcribed with random Primer (Takara), dNTP Mix (Takara) and Prime Script (Takara). Then, the amount of mRNA was quantified by performing qPCR. The Primer Set is shown in Table 1 (IL-1b: *21 (SEQ ID NOs: 19, 20) in Table 1, (MMP13: *22 (SEQ ID NOs: 21, 22) in Table 1).

### Results

### Reanalysis of scRNA-seq data in human breast cancer specimen

By integrating single-cell RNA-Seq reanalysis of human specimens, an attempt was made to identify RNA-binding proteins specifically expressed in the breast cancer stem cell fractions. ZCCHC24 was successfully identified as a novel RNA-binding protein with unknown function that is strongly expressed in cell fractions that exhibit stem cell-like expression NRP1 (+) ZEB 1 (+) EPCAM (-) (Figure 1).

### Genetic screening

The PC9-KI cell line was established in which a HiBiT tag was knocked into the C terminus of PDL1 in the lung adenocarcinoma cell line PC9. Genetic screening was performed to quantitatively measure changes in the PD-L1 protein expression by forcibly expressing 1030 types of RNA binding proteins in this PC9-KI cell line by lentiviral vector infection (Figure 2A). As a result, ZCCHC24 was identified as a gene that increases the expression of PDL1 (Figure 2B).

This increase of PDL1 expression by ZCCHC24 was more certainly confirmed by Western blotting (Figure 2C). Furthermore, in order to identify cells in which ZCCHC24 functions endogenously, the expressions among multiple cell lines were compared and the triple negative breast cancer cell line MDAMB231 was found to show high expression of ZCCHC24 (Figure 2D).

Therefore, when ZCCHC24 was knocked down using siRNA, it became clear that the expression of both PDL1 mRNA and protein decreased (Figure 2E, Figure 2F). Based on these results, the function of ZCCHC24 in triple negative breast cancer cells was analyzed.

An attempt was made to identify the fraction in which ZCCHC24 is strongly expressed, by utilizing deposit data from a report in which a single-cell RNA-Seq analysis of five triple negative breast cancer samples was carried out (Wu et al, EMBO J. 2020). When the cell fractions were classified using UMAP, it was found that they were divided into 23 clusters (Figure 3A). Among these, a tendency for ZCCHC24 to be specifically expressed in cluster 9 was revealed by drawing a violin plot (Figure 3B).

In order to further investigate the properties of the cell group that belongs to cluster 9, identification of marker genes was carried out, and it was revealed that vimentin, which greatly contributes to epithelial-mesenchymal transition (EMT) and the maintenance of cancer stem cells, was strongly expressed (Figure 3C). Therefore, when the expression distributions of cancer stem cell markers were compared, it was found that cluster 9 showed CD44 high, CD24 negative, NRP1 positive, and ZEB1 positive, indicating that ZCCHC24 is strongly expressed in the cancer stem cell-like fraction. Further, PDL1 was also expressed, suggesting a correlation with the expression of ZCCHC24 (Figure 3D).

### RNA-Seq/qPCR analysis

Furthermore, in order to identify genes whose expression changes due to ZCCHC24 by whole transcriptome analysis, RNA-Seq was performed on a control cell group and a cell group in which ZCCHC24 was knocked down with siRNA (human breast cancer cell line MDAMB231). As a result, 797 genes were identified as differentially expressed genes (DEGs) whose expression decreased. In particular, it was revealed that the expression of important genes characterizing cancer stemness in breast cancer, such as CD44, NRP1, ZEB1, and ZEB2, was significantly decreased (Figure 4 left, Figure 5A). Furthermore, it was revealed that the expression of not only PDL1 but also cytokines and chemokines important for cancer survival, such as IL6, IL8, and CXCL1, was significantly reduced (Figure 5A).

Further, it was confirmed by qPCR that the expression levels of CD44, NRP1, and ZEB1 were similarly reduced by knockdown of ZCCHC24 in breast cancer patient-derived human specimen cells (PDX) (Figure 4, right).

Therefore, when the percentage change of the breast cancer stem cell fraction defined as CD44 positive, NRP1 positive in cells in which ZCCHC24 was actually knocked down in MDAMB231 was measured using FACS, it was revealed that knockdown of ZCCHC24 reduced the ratio of the cancer stem cell fraction (Figure 5B, Figure 5C).

Furthermore, in order to demonstrate that these events are not specific to MDAMB231, knockdown of ZCCHC24 was performed in another triple negative breast cancer cell line, HCC38, and it was found that the expression of cancer stem cell-like cell surface markers such as CD44 and NRP1 was decreased at the mRNA level (Figure 5D).

Furthermore, when surface antigens were similarly analyzed by FACS, it was revealed that the CD44 positive, NRP1 positive cell fraction was significantly reduced by the knockdown of ZCCHC24 (Figure 5E, Figure 5F). These results suggested that ZCCHC24 is an important gene for maintaining the expression of a group of genes that characterize cancer stemness.

### eCLIP-Seq

In addition, eCLIP-Seq was performed on cells forced to express ZCCHC24 in order to identify the mRNA region to which ZCCHC24 directly binds as an RNA-binding protein by whole transcriptome analysis. As a result, it was revealed that ZCCHC24 targets 4821 genes, and when merged with RNA-Seq DEGs, 364 genes were identified as target genes whose expression is regulated by direct binding of ZCCHC24 (Figure 6A). Classification of the binding regions in these target genes using RSeqC revealed that they bind to the 3' UTR region and CDS (Figure 6B).

When the peak patterns of these target genes were actually analyzed, it became clear that a group of genes such as PDL1, IL6, IL8, CXCL1, CD44, and NRP1 were directly bound to the 3' UTR as target genes (Figure 6C). In addition, GO term analysis and pathway analysis of these target genes using DAVID revealed that they control a group of genes that have the functions of negatively regulating the apoptotic process and regulating cell proliferation, as well as regulate the pathways such as focal adhesion and regulation of the actin cytoskeleton (Figure 6D, Figure 6E). These results strongly suggest that ZCCHC24 is an RNA-binding protein that directly binds to and regulates the CDS and 3' UTR of genes that are important for controlling cancer stemness.

### PAR-CLIP

Furthermore, when PAR-CLIP analysis was performed on MDAMB231 with the aim of identifying the mRNA directly targeted by ZCCHC24 and its binding motif by whole transcriptome analysis, it was found that ZCCHC24 recognizes a novel unique motif sequence (A/U)GU(A /U)U(A/U)U and binds to the 3' UTR of the mRNA of a group of genes that characterize cancer stemness, such as ZEB1, CD44, and NRP1 (Figure 7).

### BRIC-Seq

In order to understand at what level the expression is regulated by ZCCHC24, BRIC-Seq was used to measure the rate of RNA degradation in MDAMB231 in which ZCCHC24 and control were knocked down with siRNA. There was a tendency toward a significant decrease in the stability of RNA of a group of genes that characterize breast cancer stemness, such as ZEB1, CD44, and NRP1 (Figure 8).

### Actinomycin D test

In order to actually investigate the contribution of ZCCHC24 to target gene mRNA stability, a test was conducted to measure the mRNA stability by stopping transcription with actinomycin D. As a result, it was found that the knockdown of ZCCHC24 reduced the mRNA stability in target genes such as CD274 (PDL1), CXCL8 (IL8), IL6, CD44, NRP1, and ZEB1 in MDAMB231 (Figure 9A). Furthermore, in HCC38, the stability of CD44 and NRP1 mRNA was similarly reduced by knockdown of ZCCHC24 (Figure 9B). These results strongly suggest that ZCCHC24 confers stability by directly binding to the mRNA of a target gene.

### Sphere formation assay

Therefore, in order to actually investigate the change in cancer stemness caused by ZCCHC24, ZCCHC24 was knocked down with siRNA in MDAMB231 and a sphere formation assay was performed, and it became clear that there was a significant decrease in the sphere formation ability by knockdown of ZCCHC24 (Figure 10A, Figure 10B).

### Extreme dilution assay (ELDA)

ELDA was performed on the triple negative breast cancer cell lines MDAMB231 and HCC38 to quantify changes in the number of cancer stem cell-like cells per cell count, and the trend became clear that the siRNA knockdown of ZCCHC24 significantly reduces the number of breast cancer stem cells per cell count compared to the control (Figure 11).

### In vivo colony formation assay

In order to evaluate tumorigenicity at the in vivo level, an in vivo tumorigenesis assay was conducted in the triple negative breast cancer cell line MDAMB231 and a subcutaneous transplantation model of PDX, and a tendency was observed in which the siRNA knockdown of ZCCHC24 significantly reduces tumorigenicity compared to the control (Figure 12). These results revealed that ZCCHC24 characterizes cancer stemness both in vitro and in vivo.

### Single cell RNA-seq analysis for the PDX subcutaneous transplantation model

In order to analyze in more detail the changes in cancer constituent cells caused by ZCCHC24 knockdown in the PDX subcutaneous transplantation model, single cell RNA-seq analysis was performed for samples obtained by subcutaneously transplanting into NOG mice cells that had been knocked down using siRNA against ZCCHC24 and control, and it revealed that the stem cell-like population exhibiting the cancer stem cell-like expression of NRP1 (+) ZEB 1 (+) CD24 (-) EPCAM (-) was significantly reduced. In particular, it was found that the stem cell-like population was largely divided into two groups of a cell population characterized by NRP1 (+) NCAM1 (+) (referred to as "Stem I") and a cell population characterized by NRP1 (+) NCAM1 (-) (referred to as "Stem II"), and in particular, the Stem II population was significantly reduced by the knockdown of ZCCHC24. This significantly reduced Stem II is a cell fraction that is positive for genes such as ZEB1 and NRP1 together with ZCCHC24, and which also expresses specific proteoglycans such as POSTN and DCN (Figure 13A).

### scRNAseq analysis of patient-derived breast cancer specimens in the NOG mouse subcutaneous transplantation model

Specimens from three triple negative breast cancer patients were subcutaneously transplanted into NOG mice, and scRNAseq analysis was performed on them. As a result, the NRP1 (+) ZEB1 (+) CD24 (-) EPCAM (-) fraction, which had previously been identified as a conventional breast cancer stem cell fraction in also specimens derived from other breast cancer patients, is largely divided into two, and among them, ZCCHC24 was identified as an RNA-binding protein that is specifically expressed in the NRP1 (+) ZEB1 (+) NCAM1 (-) fraction (defined as Stem II) (Figure 13B).

### Single cell RNA-Seq analysis of the retransplantation model

### • Analysis of tumors formed by subcutaneous PDX transplantation

When analysis of the tumor site formed by subcutaneous PDX transplantation was performed, it was found that ZCCHC24 is specifically expressed in the Stem II fraction of NRP1 (+) NCAM1 (-) (Figure 14).

### • Tumor formation assay

As a result of verifying the tumorigenicity of the sorted tumor cells, the tumorigenicity of Stem II was significantly higher compared to that of a mixture of Stem I and Stem II (Figure 15).

### • Single cell RNA-Seq analysis

Furthermore, when scRNAseq analysis was performed on samples of the Stem II retransplantation, it was again observed that in addition to the Stem II fraction, which is characterized by NRP1 (+) NCAM1 (-), there were Stem I, which is characterized by NRP1 (+) and NCAM1 (+), and an epithelial fraction, which is NRP1 (-), and it was found that it shows fractions similar to those of the tumor before sorting (Figure 16).

Thus, single-cell RNASeq analysis using the patient-derived xenografts shows that ZCCHC24 shows specifically high expression in the breast cancer stem cell fraction, which has been defined as CD44 positive, CD24 negative, NRP1 positive, and ZEB1 positive. Furthermore, single-cell RNAseq analysis using PDX revealed that the expression of ZCCHC24 was particularly high in cells of the Stem II population, which is characterized by NRP1 positive, NCAM1 negative (Figure 3D), and the cells of the Stem II population decreased (Figure 13A) and tumorigenicity was reduced (Figure 12) under conditions where ZCCHC24 was knocked down, showing that ZCCHC24 is important in maintaining the Stem II population.

Furthermore, it is shown by tumor formation assay that this Stem II population had a high tumor-forming ability when sorted and re-transplanted (Figure 15), and single-cell RNASeq analysis revealed that the tumor population established from retransplantation of this Stem II population again differentiated into various tumor cell fractions (Figure 16). Further, it has been shown that the Stem II population centered on ZCCHC24 had a high breast cancer forming ability, and in addition to ZCCHC24, the stem cell fraction that expresses ZCCHC24 can also be a therapeutic target itself.

### Compound screening for identification of ZCCHC24 expression-regulating compounds

As mentioned above, ZCCHC24 is strongly expressed in cancer stem cell-like cells, and maintains the expression of genes important for maintaining cancer stemness by directly binding to the mRNA of these genes. Therefore, in order to clarify the expression induction mechanism of ZCCHC24, labeled reporter cells were created by inserting a HiBiT tag into the C terminus of ZCCHC24, 1280 types of compounds in LOPAC (Sigma Aldrich) were administered to these reporter cells at a concentration of 1 µM for 24 hours, and compound screening was performed (Figure 17A).

As a result, when compounds with Z score > 3.0 above were defined as up-regulators and compounds with Z score < -3.0 below were defined as down-regulators, 11 up-regulators and 9 down-regulators were identified (Figure 17B).

It is noteworthy that BET inhibitors and topoisomerase inhibitors such as idarubicin, nitidine, and camptothecin, which are expected to have clinical effects on refractory breast cancer including triple negative breast cancer, have been specifically extracted as up-regulators (Figure 17C). On the other hand, microtubule polymerization inhibitors and Wee1/Chk1 inhibitors, which are important in the DNA damage response pathway, were identified as down-regulators (Figure 17D). Therefore, in view of the possibility that the DNA damage response pathway is particularly important in controlling the expression of ZCCHC24, it was decided to focus on BET inhibitors as up-regulators and Wee1/Chk1 inhibitors as down-regulators.

Therefore, in measurement of the EC₅₀ concentration for the maximal increase in the ZCCHC24 expression using JQ1, a typical BET inhibitor, it was found to be about 50 nM, indicating that it indeed increases the ZCCHC24 expression (Figure 18A). On the other hand, the IC₅₀ of JQ1 in MDAMB231 for maximal cell survival inhibition was about 0.1 µM (Figure 18B).

The transcription factor ZEB1 received attention as the mechanism by which this BET inhibitor dramatically increases the expression of ZCCHC24. ZEB1 is a transcription factor whose importance in EMT and drug resistance has been strongly suggested in breast cancer, lung cancer and such, and it has also been identified as a target gene of ZCCHC24, as shown in Figures 6 and 7. Therefore, when referring to the histone marker ChIP database and past ChIP-Seq data by ZEB 1, it was found that there is a region between the first and second exon where the histone markers of H3K4Me1 and H3K27Ac show peaks, and the peak of ZEB1 is also observed (Figure 18C). Moreover, the expression of ZEB1 was dramatically increased by administering JQ1 (Figure 18D).

Therefore, when the dependence of ZCCHC24 on ZEB1 due to JQ1 was verified by siRNA knockdown, the increase in the expression of ZCCHC24 due to JQ1 was significantly reduced by knockdown of ZEB1 (Figure 18E).

These results strongly suggest that BET inhibitors increase the expression of ZCCHC24 through increased expression of ZEB 1. Furthermore, compounds that control the axis of ZEB1-ZCCHC24 are of great significance in reducing drug resistance caused by BET inhibitors. Therefore, a focus was placed on Wee1/Chk1 inhibitors as a result of compound screening. The EC₅₀ concentration of PD407824, a Wee1/Chk1 inhibitor, for maximally suppressing the expression of ZCCHC24 was measured and was approximately 50 nM (Figure 19A). On the other hand, although a certain degree of cell activity was suppressed by PD407824, the cell activity was not suppressed to the extent that the IC₅₀ could be calculated (Figure 19B).

Furthermore, when JQ1 and PD407824 were administered individually, no decrease in cancer stem cell-like populations was observed when JQ1 was administered, whereas a tendency for the cell population to decrease was observed when PD407824 was administered. Furthermore, by the combined administration of JQ1 and PD407824, a decrease in the cancer stem cell-like population was observed (Figure 19C, Figure 19D).

Based on these tendencies, the cell proliferation inhibitory effect of the combined use of 100 nM JQ1 and 1 µM PD407824 was investigated, and it was found that the combined use certainly had an additive effect (Figure 19E).

### ZCCHC24 knockout mice show resistance to LPS shock

ZCCHC24 knockout mice were established using the CRISPR-Cas9 system. When ZCCHC24 knockout mice were intraperitoneally injected with LPS at a ratio of 15 mg/kg, the survival period after LPS injection was prolonged compared to wild type mice. It is suggested that ZCCHC24 is important as an RNA binding protein that controls inflammation (Figure 20).

### ZCCHC24 knockout mice show resistance to high-fat diet loading

A high fat diet (HFD) was fed to ZCCHC24 knockout mice. As a result, a significant suppression in weight gain was observed in ZCCHC24 knockout mice compared to wild type mice approximately three months after the start of loading. It has been shown that ZCCHC24 may also play an important role in obesity (Figure 21).

### Novel nucleic acid preparation targeting the RNA sequence targeted by ZCCHC24

By using the designed microRNA-mimic preparation, an attempt was made to broadly and simultaneously control breast cancer stemness genes and inflammatory genes targeted by ZCCHC24.

When a mixture of mimic-1 (miR-PBE1), mimic-2 (miR-PBE2), and mimic1,2 (miR-mix) was introduced into the MDAMB231 cell line, it was found that the expression of genes such as ZEB1, IL-6, ITGB1, and THBS1, which are necessary for breast cancer stemness and cancer cell invasion, was suppressed (Figure 22, Figure 23).

Furthermore, when a mixture of mimic-1 (miR-PBE1), mimic-2 (miR-PBE2), and mimic1,2 (miR-mix) was introduced into breast cancer TNBC patient-derived cells (PDX), the expression of genes such as IL-6, CD44, ITGB1, and NRP1, which are similarly necessary for breast cancer stemness and cancer cell invasion, was suppressed (Figure 24).

Furthermore, when mimic1 (miR-PBE1) was introduced into the human chondrocyte cell line SW1353, the expression of MMP13 and IL1-β, which are important effector molecules in knee osteoarthritis, was suppressed (Figure 25).

These results demonstrated in vitro that a novel mimic RNA preparation targeting the RNA sequence bound by ZCCHC24 may have therapeutic effects for cancer and knee osteoarthritis.

Table 1 below shows the sequence symbols, sequence names, sequences, and sequence numbers in the attached sequence listing used in the examples.

**Table 1**

| Symbol | Sequence name | Sequence | Sequence No. in sequence listing |
|---|---|---|---|
| *1 | ZCCHC24 binding target sequence | WGUWHWWA | |
| *2 | ZCCHC24 binding target sequence | UGUAHAWA | |
| *3 | ZCCHC24 binding target sequence | WGUWUWUA | |
| *4 | siRNA negative control | Stealth RNAi^{™} siRNA Negative Control Hi GC,Thermofisher Scientific | |
| *5 | siRNA ZCCHC24 | StealthRNA HSS137253, Thermofisher Scientific | |
| *6 | ZCCHC24 qPCR primer set | CCAACCTTCTGGTTGAGCTG, CATCCAGGGAAAACAACACAGA | SEQ ID NO: 1 |
| | | | SEQ ID NO: 2 |
| *7 | ZEB1 qPCR primer set | GATGACCTGCCAACAGACCA, GTCCTCTTCAGGTGCCTCAG | SEQ ID NO: 3 |
| | | | SEQ ID NO: 4 |
| *8 | CD44 qPCR primer set | AACTGGAACCCAGAAGCACA, CAGCTGTCCCTGTTGTCGAA | SEQ ID NO: 5 |
| | | | SEQ ID NO: 6 |
| *9 | NRP1 qPCR primer set | CGGGACCCATTCAGGATCAC, CGGGACCCATTCAGGATCAC | SEQ ID NO: 7 |
| | | | SEQ ID NO: 8 |
| *10 | PSMB2 qPCR primer set | TTCCCTCTGAGGTGCTGTCT, ACATCTTGTCATGATCGTCCTTCA | SEQ ID NO: 9 |
| | | | SEQ ID NO: 10 |
| *11 | eCLIP/PAR-CLIP RNA adapter | AGAUCGAAGAGCGUCGUGUAG | SEQ ID NO: 11 |
| *12 | eCLIP/PAR-CLIP reverse transcription primer | ACACGACGCTCTTCCGA | SEQ ID NO: 12 |
| *13 | eCLIP/PAR-CLIP rand103Tr3 linker | 5Phos/NNNNNNNNNNAGATCGGAAGAG CACACGTCTG/3SpC3 | SEQ ID NO: 13 |
| *14 | crRNA ZCCHC24 C terminus | ACTGCCGTCGCGTGCAGTGA | SEQ ID NO: 14 |
| *15 | Oligo DNA ZCCHC24 HiBiT | AGAGCAAGGAGCACCCGCAGCACCTCT GCGAGAAGTGCAAGGTCCTGGGCTACT ACTGCCGTCGCGTGCAGGACTACAAGG ACGACGACGACAAGGTGAGCGGCTGG CGGCTGTTCAAGAAGATTAGCTGACGG GCTGCCCGCCCGCACCCAGAGCCACC CCCCGCCAGCCCGAGGAGACGCTGCT TCCCTGTGCTACTC | SEQ ID NO: 15 |
| *16 | KO mouse generation CRISPR-Cas9 gRNA | GGUAGACCCAGUUGAGUAGC | SEQ ID NO: 16 |
| *17 | Seed target sequence 1 | UGUAUAU | |
| *18 | Seed target sequence 2 | UGUACAU | |
| *19 | miR-PBE1 | AAUAUACAUCCUCCGGGAUCCA | SEQ ID NO: 17 |
| *20 | miR-PBE2 | AUUAUACAUCCUCCGGGAUCCA | SEQ ID NO: 18 |
| *21 | IL-lb qPCR primer set | ATGATGGCTTATTACAGTGGCAA GTCGGAGATTCGTAGCTGGA | SEQ ID NO: 19 |
| | | | SEQ ID NO: 20 |
| *22 | MMP13 qPCR primer set | ACTGAGAGGCTCCGAGAAATG GAACCCCGCATCTTGGCTT | SEQ ID NO: 21 |
| | | | SEQ ID NO: 22 |

Although preferred embodiments of the invention are shown herein, it is clear to those skilled in the art that such embodiments are provided merely for illustrative purposes, and those skilled in the art may make various variations, changes, and substitutions without departing from the invention. It should be understood that various alternative embodiments of the invention described herein may be used in practicing the invention. In addition, the contents of all publications, including patents and patent application documents referenced herein, should be construed to be incorporated by reference as if they were the same as those specified herein.

### Industrial Applicability

Although many molecular targeted drugs including CDK4/6 inhibitors have been used so far to treat refractory breast cancer including triple negative breast cancer, effective treatment has not been fully established. In these refractory breast cancers, ZCCHC24 is an important gene to explain the resistance to treatment because it up-regulates the expression of cancer-promoting genes by post-transcriptional regulation, and thus has utility as a therapeutic target. Further, the present inventors have also succeeded in identifying a new breast cancer stem cell fraction that highly expresses ZCCHC24 and a cell surface marker that defines it, and this cancer stem cell fraction that expresses ZCCHC24 itself may be a potential therapeutic target. Furthermore, the present inventors have succeeded in identifying compounds that reduce the expression of ZCCHC24, and the use of such compounds may reduce drug resistance to existing treatments for refractory breast cancer. In addition, ZCCHC24 is useful as a therapeutic target not only for breast cancer, including triple negative breast cancer, but also for inflammatory diseases and obesity.

## Claims

1. A composition for use in the treatment of triple negative breast cancer, comprising an inhibitor of the ZCCHC24 protein or an agent that suppresses expression of the ZCCHC24 gene as an active ingredient for use in the treatment of a patient having a NRP1 (+) NCAM1 (-) cell fraction.

2. The composition for use in the treatment of triple negative breast cancer according to claim 1, wherein the agent that suppresses the expression of the ZCCHC24 gene is a Wee1/Chk1 inhibitor.

3. The composition for use in the treatment of triple negative breast cancer according to claim 1 or 2, for use in combination with a BET inhibitor.

4. The composition for use in the treatment of triple negative breast cancer according to claim 1, wherein the inhibitor of the ZCCHC24 protein is selected from the group consisting of neutralizing antibodies, nucleic acid drugs, and low molecular weight compounds.

5. The composition for use in the treatment of triple negative breast cancer according to claim 2, wherein the Wee1/Chk1 inhibitor is selected from the group consisting of PD407824, AZD-1775, ZN-c3, Debio-0123, IMP-7068, GDC-0575, ESP-01, PNT-737, BEBT-260, AZD7762, LY2603618, MK- 8776, CHIR-124, PF-477736, rosovitin, SNS-032, dinaciclib, flavopiridol, AT7519, purvalanol A, RO-3306, SU9516, XL413, NU6027, P276-00, AZD5438, PHA-793887, JNJ-7706621 , BMS-265246, milciclib, MK-8776, R547, and adavosertib.

6. The composition for use in the treatment of triple negative breast cancer according to claim 1, further comprising a BET inhibitor.

7. The composition for use in the treatment of triple negative breast cancer according to claim 6, wherein the BET inhibitor is selected from the group consisting of JQ1, pelabresib, BMS-986158, INCB-057643, ODM-207, PLX-2853, ABBV-744, BI-894999, BPI-23314, CC-90010, FT-1101, JAB- 8263, mibebresib, SF-1126, and SYHA-1801.

8. The composition for use in the treatment of triple negative breast cancer according to claim 5, wherein the Wee1/Chk1 inhibitor is selected from the group consisting of PD407824, AZD-1775, ZN-c3, Debio-0123, IMP-7068, GDC-0575, ESP-01, PNT-737, BEBT-260, AZD7762, LY2603618, MK- 8776, CHIR-124, PF-477736, rosovitin, SNS-032, dinaciclib, flavopiridol, AT7519, purvalanol A, RO-3306, SU9516, XL413, NU6027, P276-00, AZD5438, PHA-793887, JNJ-7706621 , BMS-265246, MK-8776, and R547.

9. The composition for use in the treatment of triple negative breast cancer according to claim 1, wherein the agent that suppresses the expression of the ZCCHC24 gene is a nucleic acid drug.

10. The composition for use in the treatment of triple negative breast cancer according to claim 1, wherein the inhibitor of the ZCCHC24 protein comprises a 19-25 base long miRNA having a sequence with a full-length GC content of 40-60%, and consists of:
(i) a seed sequence complementary to at least a portion of WGUWHWWA;
(ii) an adenine or a uracil base on the 5' side of the seed sequence; and
(iii) a scrambled sequence on the 3' side of the seed sequence.

11. A composition for use in the treatment of triple negative breast cancer, comprising as an active ingredient an inhibitor of the ZCCHC24 protein or an agent that suppresses the expression of theZCCHC24 gene (with the proviso that milciclib and adavosertib are excludded).

12. A composition for use in the treatment of triple negative breast cancer, comprising as an active ingredient an inhibitor of the ZCCHC24 protein or a nucleic acid drug that suppresses the expression of the ZCCHC24 gene.

13. A composition for use in the treatment of triple negative breast cancer to be used in combination with a BET inhibitor, comprising an inhibitor of the ZCCHC24 protein or an agent that suppresses the expression of the ZCCHC24 gene as an active ingredient.

14. A composition for use in the treatment of triple negative breast cancer, comprising as an active ingredient an inhibitor of the ZCCHC24 protein or an agent that suppresses the expression of the ZCCHC24 gene, wherein the agent that suppresses the expression of the ZCCHC24 gene is a Wee1/Chk1 inhibitor, and wherein the Wee1/Chk1 inhibitor is selected from the group consisting of PD407824, AZD-1775, ZN-c3, Debio-0123, IMP-7068, GDC-0575, ESP-01, PNT-737, BEBT-260, AZD7762, LY2603618, MK-8776, CHIR-124, PF-477736, rosovitin, SNS-032, dinaciclib, flavopiridol, AT7519, purvalanol A, RO-3306, SU9516, XL413, NU6027, P276-00, AZD5438, PHA-793887, JNJ-7706621, BMS-265246, MK-8776, and R547.

15. A method for testing breast cancer in a test subject, comprising:
a) measuring the amount of the ZCCHC24 protein in a body fluid sample derived from the test subject; and
b) comparing the amount of the ZCCHC24 protein with a predetermined reference value, wherein the test method indicates the possibility of breast cancer based on that the amount of the ZCCHC24 protein is higher than the predetermined reference value.

16. A method for testing breast cancer in a test subject, comprising determining whether or not the cell sample derived from the test subject has a NRP1 (+) NCAM1 (-) cell fraction, wherein the test method indicates the possibility of breast cancer based on the presence of the subcellular fraction.

17. A method for examining the condition of breast cancer in a test subject, comprising determining whether or not the cell sample derived from the test subject has a NRP1 (+) NCAM1 (-) cell fraction, wherein the test method determines that the breast cancer has a high degree of malignancy when it has the subcellular fraction.

18. A method for examining the condition of breast cancer in a test subject, comprising determining whether or not the cell sample derived from the test subject has a NRP1 (+) NCAM1 (-) cell fraction, wherein the test method identifies the test subject to be eligible for administration of a composition for use in the treatment of triple negative breast cancer when it has the cell fraction.

19. A composition for use in the treatment of an inflammatory disease, comprising as an active ingredient an inhibitor of the ZCCHC24 protein or an agent that suppresses the expression of the ZCCHC24 gene.

20. The composition for use in the treatment of an inflammatory disease according to claim 19, wherein the agent that suppresses the expression of the ZCCHC24 gene is a Wee1/Chk1 inhibitor.

21. The composition for use in the treatment of an inflammatory disease according to claim 19, wherein the agent that suppresses the expression of the ZCCHC24 gene is a nucleic acid drug.

22. The composition for use in the treatment of an inflammatory disease according to claim 19, wherein the inhibitor of the ZCCHC24 protein comprises a 19-25 base long miRNA having a sequence with a full-length GC content of 40-60%, and consists of:
(i) a seed sequence complementary to at least a portion of WGUWHWWA;
(ii) an adenine or a uracil base on the 5' side of the seed sequence; and
(iii) a scrambled sequence on the 3' side of the seed sequence.

23. A composition for use in the treatment of obesity, comprising as an active ingredient an inhibitor of the ZCCHC24 protein or an agent that suppresses the expression of the ZCCHC24 gene.

24. The composition for use in the treatment of obesity according to claim 23, wherein the agent that suppresses the expression of the ZCCHC24 gene is a Wee1/Chk1 inhibitor.

25. The composition for use in the treatment of obesity according to claim 23, wherein the agent that suppresses the expression of the ZCCHC24 gene is a nucleic acid drug.

26. The composition for use in the treatment of obesity according to claim 23, wherein the inhibitor of the ZCCHC24 protein comprises a 19-25 base long miRNA having a sequence with a full-length GC content of 40-60%, and consists of:
(i) a seed sequence complementary to at least a portion of WGUWHWWA;
(ii) an adenine or a uracil base on the 5' side of the seed sequence; and
(iii) a scrambled sequence on the 3' side of the seed sequence.

27. A method of screening for agents for use in the treatment of triple negative breast cancer, inflammatory disease, or obesity, wherein the method comprises:
(i) a step of preparing cells expressing ZCCHC24;
(ii) a step of contacting a test substance with the cells;
(iii) a step of measuring the expression level of ZCCHC24; and
(iv) a step of selecting a substance that reduces the expression level of ZCCHC24.

28. A method for treating and/or preventing triple negative breast cancer, inflammatory disease, or obesity in a subject in need thereof, wherein the method comprises administering to the subject an effective amount of an inhibitor of the ZCCHC24 protein or an agent that suppresses the expression of the ZCCHC24 gene.

29. Use of an inhibitor of the ZCCHC24 protein or an agent that suppresses the expression of the ZCCHC24 gene in the manufacture of a medicament for the treatment and/or prevention of triple negative breast cancer, inflammatory disease, or obesity.
